(19) Europäisches Patentamt
European Patent Office
Office européen des brevets

(11) **EP 4 050 612 B1**

(12) **EUROPEAN PATENT SPECIFICATION**

(45) Date of publication and mention
of the grant of the patent:
**03.12.2025   Bulletin 2025/49**

(21) Application number: **20878286.2**

(22) Date of filing: **20.10.2020**

(51) International Patent Classification (IPC):
***G16C 20/50*** (2019.01)          ***G16C 20/70*** (2019.01)

(52) Cooperative Patent Classification (CPC):
**G16C 20/50;** G16C 20/70

(86) International application number:
**PCT/KR2020/014354**

(87) International publication number:
**WO 2021/080295 (29.04.2021 Gazette 2021/17)**

(54) **METHOD AND DEVICE FOR DESIGNING COMPOUND**

VERFAHREN UND VORRICHTUNG ZUM ENTWURF EINER VERBINDUNG

PROCÉDÉ ET DISPOSITIF DE CONCEPTION DE COMPOSÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.10.2019   KR 20190130769**
**16.04.2020   KR 20200046192**

(43) Date of publication of application:
**31.08.2022   Bulletin 2022/35**

(73) Proprietor: **Standigm Inc.**
**Seoul 06234 (KR)**

(72) Inventors:
• YOO, Ji Ho
Hwaseong-si, Gyeonggi-do 18445 (KR)
• HA, Min Kyu
Seoul 08259 (KR)
• CHAE, Jong Hwan
Seoul 05266 (KR)
• SON, Chi Won
Yongin-si, Gyeonggi-do 16874 (KR)
• JIN, Sang Hyung
Seoul 06127 (KR)
• SHIN, Jae Hong
Seongnam-si, Gyeonggi-do 13567 (KR)
• KIM, Han Jo
Yongin-si, Gyeonggi-do 16927 (KR)
• KIM, Si U
Seongnam-si, Gyeonggi-do 13502 (KR)
• SONG, Sang Ok
Seongnam-si, Gyeonggi-do 13436 (KR)

(74) Representative: **Jung, Minkyu**
**Wuesthoff & Wuesthoff**
**Patentanwälte und Rechtsanwalt PartG mbB**
**Schweigerstraße 2**
**81541 München (DE)**

(56) References cited:
**KR-A- 20190 087 898**

• CHUNQUAN SHENG ET AL: "Fragment
Informatics and Computational Fragment-Based
Drug Design: An Overview and Update",
MEDICINAL RESEARCH REVIEWS, vol. 33, no. 3,
19 May 2013 (2013-05-19), US, pages 554 - 598,
XP055335769, ISSN: 0198-6325, DOI: 10.1002/
med.21255
• FANG-YU LIN ET AL: "Structure-Based Fragment
Hopping for Lead Optimization Using Predocked
Fragment Database", JOURNAL OF CHEMICAL
INFORMATION AND MODELING, vol. 51, no. 7, 25
July 2011 (2011-07-25), US, pages 1703 - 1715,
XP055415546, ISSN: 1549-9596, DOI: 10.1021/
ci200136j
• MIKKO J. VAINIO ET AL: "Scaffold Hopping by
Fragment Replacement", JOURNAL OF
CHEMICAL INFORMATION AND MODELING, vol.
53, no. 7, 11 July 2013 (2013-07-11), US, pages
1825 - 1835, XP055752416, ISSN: 1549-9596, DOI:
10.1021/ci4001019

EP 4 050 612 B1

- STÅHL NICLAS, FALKMAN GÖRAN, KARLSSON ALEXANDER, MATHIASON GUNNAR, BOSTRÖM JONAS: "Deep Reinforcement Learning for Multiparameter Optimization in de novo Drug Design", JOURNAL OF CHEMICAL INFORMATION AND MODELING, vol. 59, no. 7, 19 June 2019 (2019-06-19), pages 3166 - 3176, XP055803313, ISSN: 1549-9596, DOI: 10.1021/ acs.jcim.9b00325
- KAWAI KENTARO, NAGATA NAOYA, TAKAHASHI YOSHIMASA: "De novo design of drug-like molecules by a fragment-based molecular evolutionary approach", JOURNAL OF CHEMICAL INFORMATION AND MODELING, vol. 54, no. 1, 28 December 2013 (2013-12-28), pages 49 - 56, XP055805856, ISSN: 1549-9596, DOI: 10.1021/ci400418c
- BORIS SATTAROV, IGOR I. BASKIN, DRAGOS HORVATH, GILLES MARCOU, ESBEN JANNIK BJERRUM, ALEXANDRE VARNEK: "De novo molecular design by combining deep autoencoder recurrent neural networks with generative topographic mapping", JOURNAL OF CHEMICAL INFORMATION AND MODELING, vol. 59, no. 3, 20 February 2019 (2019-02-20), pages 1182 - 1196, XP055731747, ISSN: 1549-9596, DOI: 10.1021/acs.jcim.8b00751
- JUSTIN GILMER, SAMUEL S. SCHOENHOLZ, PATRICK F. RILEY, ORIOL VINYALS, GEORGE E. DAHL: "Neural message passing for quantum chemistry", COMPUTER SCIENCE, 4 April 2017 (2017-04-04), pages 1 - 14, XP055700744

## Description

## Technical Field

This disclosure relates to a method and an apparatus for designing a molecular structure of a new drug candidate.

In addition, in a method of automatically designing a molecular structure of a new drug candidate, this disclosure relates to a method and an apparatus of designing a more improved structure by selectively modifying a desired part of an existing structure.

## Background Art

Recently, in the field of new drug development, a molecular structure designing method using deep learning based artificial intelligence (AI) technology has been actively used to discover new drug candidates. Existing molecular structure designs were done by skilled professionals, and showed clear logical rationale and relatively high accuracy. However, there was a limit to quickly carrying out large amount of design work. Regarding this, AI-based design is a method that may automatically design a structure of a candidate material from data and gradually improve performance by adding post-evaluation results for a structure of a designed material to the data. It has the advantage of quickly and systematically developing candidate materials using a large amount of data compared to existing methods that rely on the experiences of the skilled experts.

However, since existing AI-based molecular structure designing methods aim to generate a new "complete" molecular structure, it is difficult to apply to the design of a method of changing only an interesting "part" of a molecular structure. Specifically, when a search for a new drug candidate material is in progress, potent candidate materials showing good drug efficacy may be discovered, and which part (structure) of the potent materials is important for exhibiting a target drug effect may be determined. In this case, a partial structure of a potent material may be actively utilized as important information when further generating a candidate material. However, existing AI-based molecular structure designing methods do not have a method for fixing partial structures of a molecular structure so as not to change it, so it is not easy to apply a partial structure of a potent material to the generation of a candidate material, and even if the partial structure of a potent material is applied to the generation of a candidate material, there was problems in that efficiency is greatly reduced as a number of unwanted structures are generated. The document ("Deep Reinforcement Learning for Multiparameter Optimization in de novo Drug Design", vol. 59, no. 7, 19 June 2019, pages 3166-3176) outlines a fragment-based reinforcement learning approach based on an actor-critic model, for the generation of novel molecules with optimal properties. In the document it is described that the actor and the critic are both modeled with bidirectional long short-term memory (LSTM) networks, the AI method learns how to generate new compounds with desired properties by starting from an initial set of lead molecules and then improving these by replacing some of their fragments, and a balanced binary tree based on the similarity of fragments is used in the generative process to bias the output toward structurally similar molecules. The document ("De novo design of drug-like molecules by a fragment-based molecular evolutionary approach", vol. 54, no. 1, 28 December 2013, pages 49-56) outlines a similarity-driven simple evolutionary approach to produce candidate molecules of new drugs. In the document it is described that the aim of the method is to explore the candidates that are structurally similar to the reference molecule and yet somewhat different in not only peripheral chains but also their scaffolds, the method employs a known active molecule of our interest as a reference molecule which is used to navigate a huge chemical space, the reference molecule is also used to obtain seed fragments, an initial set of individual structures is prepared with the seed fragments and additional fragments using several connection rules, the fragment library is preferably prepared from a collection of known molecules related to the target of the reference molecule, every fragment of the library can be used for fragment-based mutation, and all the fragments are categorized into three classes; rings, linkers, and side chains. The document ("Fragment Informatics and Computational Fragment-Based Drug Design: An Overview and Update", vol. 33, no. 3, 19 May 2013, pages 554-598) outlines analysis of complementarity between computational and experimental FBDD and algorithms and successful examples of their applications. In the document fragment-based cheminformatics tools, high-throughput fragment docking, and fragment-based de novo drug design are disclosed.

## Disclosure of Invention

## Technical Goals

An embodiment in this document provides a method that is one of AI-based molecular structure designing methods and an apparatus, and the method and the apparatus relate to automatically designing a molecular structure of a new drug candidate by selectively modifying only a part of a given material structure.

## Technical solutions

The invention is defined by the appended claims.

## Effects

According to example embodiments, provided is a system of designing a new compound through AI learning using existing database, and with respect to the generated compound, the system may more easily generate a hit compound in consideration of the difficulty of synthesis, the degree of similarity to an existing molecular structure, and compatibility with a target protein.

According to example embodiments, provided is, through the use of the AI-based molecular structure designing method, effect of accelerating search for new drug candidates in various fields such as further improvement of potential candidates as well as in the discovery stage of new candidate materials.

According to example embodiments, by dividing a source molecular structure into partial structures, and modifying and applying only a partial structure, a new material having an action similar to that of the source molecular structure may be easily generated.

**Brief Description of Drawings**

FIG. 1 is a diagram for explaining a method of generating a compound according to example embodiments of the present disclosure.

FIG. 2 is a diagram for explaining specific elements related to a method of generating a compound according to example embodiments of the present disclosure.

FIG. 3 is a diagram for explaining a compound discovery phase according to example embodiments of the present disclosure.

FIG. 4 is a diagram for explaining a compound generation according to example embodiments of the present disclosure and an analysis process according thereto.

FIG. 5 is a diagram for explaining a relationship between a latent space and an original structure space for compound generation according to example embodiments of the present disclosure.

FIG. 6 is a diagram for explaining procedures performed in a process of generating a molecule according to example embodiments of the present disclosure.

FIG. 7 is a block diagram for explaining a computing apparatus in which a method according to example embodiments of the present disclosure is executed.

FIG. 8 is a conceptual diagram illustrating a process of discovering a candidate molecule.

FIG. 9 is a conceptual diagram illustrating an operation of a new drug discovery platform according to example embodiments of the present disclosure.

FIG. 10 is a conceptual diagram illustrating a configuration and workflow of a new drug discovery platform according to example embodiments of the present disclosure.

FIG. 11 is a diagram illustrating an example embodiment of configuring a partial structure tree.

FIG. 12 is a graph showing pattern distribution of a partial structure modification of the sampled molecule of FIG. 11 in the lead compound optimization.

FIG. 13 is a diagram schematically illustrating a molecular structure designing method according to example embodiments of the present disclosure.

FIG. 14 is a diagram illustrating a process of decomposing a molecular structure into partial structures according to example embodiments of the present disclosure.

FIG. 15 is a diagram illustrating a partial structure tree according to an example embodiment.

FIG. 16 is a diagram illustrating an encoder-decoder model according to an example embodiment.

FIG. 17 is a diagram illustrating an encoder-decoder model for further outputting topology information and molecular weight information of a partial structure according to an example embodiment.

FIG. 18 is a flowchart of a method of modifying a partial structure of a molecular structure according to an example embodiment.

FIG. 19 is a diagram illustrating an example embodiment in which a source molecule to be modified and a partial structure are input.

FIG. 20 is a diagram illustrating a partial charge scaffold for an exemplary partial structure.

FIG. 21 is a diagram illustrating a case that may come out in the process of recombination of a new partial structure.

FIG. 22 is a flowchart illustrating an encoder model learning a complete structure and a partial structure according to an example embodiment.

FIG. 23 is a diagram illustrating message information of a partial structure.

FIG. 24 is a flowchart illustrating a method of generating compound information according to an example embodiment.

FIG. 25 is a diagram schematically illustrating a system for generating compound information according to an example embodiment.

FIG. 26 is a block diagram illustrating a computing apparatus for generating compound information according to an example embodiment.

**Best Mode for Carrying Out the Invention**

Hereinafter, example embodiments of the present disclosure will be described in detail with reference to the accompanying drawings.

In describing the example embodiments, descriptions of technical contents that are well known in the technical field to which the present disclosure pertains and that are not directly related to the present disclosure will be omitted. This is to more clearly convey the gist of the present disclosure without obscuring the gist of the present disclosure by omitting unnecessary description.

For the same reason, some elements are exaggerated, omitted or schematically illustrated in the accompanying drawings. In addition, the size of each element does not fully reflect the actual size. In each figure, the same or corresponding elements are assigned the same reference numerals.

Advantages and features of the present disclosure, and a method of achieving the advantages and the features will become apparent with reference to the example embodiments described below in detail together with the accompanying drawings. However, the present disclosure is not limited to the example embodiments disclosed below, and may be implemented in various different forms. The example embodiments are provided only so that the present disclosure to be complete, and completely inform the scope of the present disclosure to those of ordinary skill in the art to which the present disclosure pertains. The present disclosure is only defined by the scope of the claims. Like reference numerals refer to like elements throughout.

At this time, it will be understood that each block of a flowchart diagram and a combination of the flowchart diagrams may be performed by computer program instructions. The computer program instructions may be embodied in a processor of a general-purpose computer or a special purpose computer, or may be embodied in a processor of other programmable data processing equipment. Thus, the instructions, executed via a processor of a computer or other programmable data processing equipment, may generate a part for performing functions described in the flowchart blocks. To implement a function in a particular manner, the computer program instructions may also be stored in a computer-usable or computer-readable memory that may direct a computer or other programmable data processing equipment. Thus, the instructions stored in the computer usable or computer readable memory may be produced as an article of manufacture containing an instruction part for performing the functions described in the flowchart blocks. The computer program instructions may be embodied in a computer or other programmable data processing equipment. Thus, a series of operations may be performed in a computer or other programmable data processing equipment to create a computer-executed process, and the computer or other programmable data processing equipment may provide steps for performing the functions described in the flowchart blocks.

Additionally, each block may represent a module, a segment, or a portion of code that includes one or more executable instructions for executing a specified logical function(s). It should also be noted that in some alternative implementations the functions recited in the blocks may occur out of order. For example, two blocks shown one after another may be performed substantially at the same time, or the blocks may sometimes be performed in the reverse order according to a corresponding function.

At this time, the term "...part" used in example embodiments indicates software or hardware components such as FPGA or ASIC, and "... part" performs certain roles. However, "...part" is not limited to software or hardware. "...part" may be configured to be in an addressable storage medium or may be configured to execute one or more processors. Therefore, as an example, "...part" may include software configurations, object-oriented software configurations, class configurations, task configurations, processes, functions, properties, procedures, subroutines, segments of program code, drivers, firmware, microcode, circuitry, data, database, data structures, tables, arrays and variables. Functions provided in the configurations and "...part" may be combined into a smaller number of configurations and "...parts" or further separated into additional configurations and "...parts." In addition, configurations and "...part" may be implemented to play one or more CPUs in an apparatus or secure multimedia card.

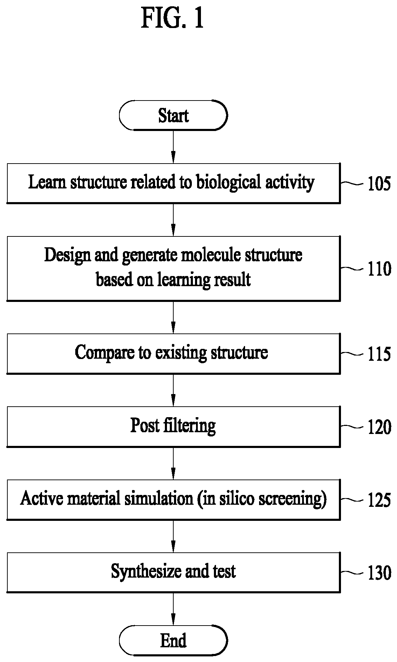

FIG. 1 is a diagram for explaining a method of generating a compound.

Referring to FIG. 1, a method for compound generation and validation is shown. At least a part of steps may be implemented in a computing apparatus. Rest of the parts may be implemented as an actual experiment, or may be implemented by inputting result information performed as an experiment into a computing apparatus.

The computing apparatus may learn about a molecular structure related to biology activity in operation 105. This may be performed based on at least one of structures and properties of a previously collected molecule. The learning may be performed by AI, and the method may be implemented in various ways.

The computing apparatus may design a molecular structure based on the learning result, and may generate a designed molecular structure in operation 110. In an example embodiment, molecule structure generation may include generating a structure of a designed molecule on the computing apparatus for simulation. In this case, factors considered for the molecule structure generation may include at least one of difficulty in synthesis, similarity to an existing molecular structure, and suitability related to a target protein.

The computing apparatus may compare the molecule structure generated in this way to a known molecular structure in operation 115. Through the procedure, the computing apparatus may determine a molecule having a novel structure among generated molecules. In this operation, the newly generated molecular structure and the existing molecular structure are determined for similarity, and when the similarity is greater than a specific value, it may be determined as not a new molecule.

In operation 120, the computing apparatus may perform post filtering on molecules having a similarity of less than or equal to a specific value to the existing molecular structure in the previous step. It may be a procedure for selecting a hit compound from among the compounds which generated by post filtering. In an example, for post filtering, at least one of docking molecule and optimizing a plurality of parameters may be performed. Further, in an example, the post filtering may be performed in consideration of synthesis difficulty. Through the procedure, a hit compound may be substantially selected. Information for the post filtering may include at least one of information associated with receiving an experimental result value and information input by a user, and according to an example embodiment, the post filtering may be performed based on an existing learning result. In this way, the number of molecules to be simulated later may be adjusted through the post filtering. In an example embodiment, the post filtering may be performed by additionally considering information associated with a main part and a secondary part of a corresponding material, and restriction on property change depending on a structure. In addition, property change restriction may be performed based on information associated with upper and lower limit values of numerical change for a specific property.

The computing apparatus may perform a simulation on the post-filtered molecules in operation 125. In an example, a simulation may be performed through in silico screening process. Through this process, by minimizing the lead optimization procedure, the number of final test target molecules may be controlled. Molecules remaining after the post filtering may be classified according to priority by a predetermined method, and subsequent steps may proceed based on the classified priorities.

Molecules selected according to the simulation result may be synthesized and tests for the properties of the synthesized molecules may be performed in operation 130. Such a procedure may be performed in a real laboratory, or may be performed in the form of deriving a final result value by inputting experimental result information into the computing apparatus.

Based on the information learned in this way, a structure for a candidate molecule is designed and generated, it is compared to an existing structure thereby determining a new molecule, it is filtered according to a desired property thereby reducing the number of target molecules, filtered molecules are simulated and a property thereof may be determined, and a molecule having the desired property is effectively produced by synthesizing and testing a material having the desired property.

Further, in an example, parameters underlying the molecular structure design and post filtering may be at least partially similar, but the property of parameter application may vary. In order to design a molecular structure more specifically, a parameter may be applied in a direction in which a wider range of property is encompassed, and in the case of filtering, a parameter may be applied to narrow the range of the desired property and to perform scoring according to the degree to which a generated molecule corresponds to the desired property.

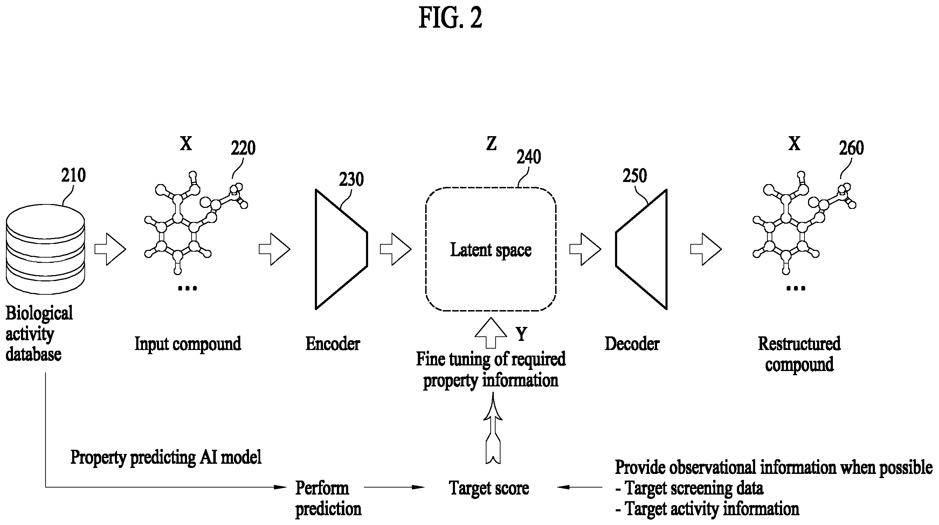

FIG. 2 is a diagram for explaining specific elements related to a method of generating a compound.

FIG. 2 illustrates a method of generating a restructured compound by selecting a specific compound based on database in which biological activity-related information is collected, using it as an input compound, and performing tuning based on a property value required in a latent space. The procedure may be performed to derive a material having a new structure.

Material-related information may be determined in the biological activity-related database in operation 210.

An input compound may be determined based on at least one of a target protein and desired property information in operation 220. The input compound may be determined based on existing compound information, and may be selected based on at least one of an action relationship with a target protein and the property information.

An encoding operation may be performed to change information related to the input compound into a latent space in operation 230.

The computing apparatus may design a desired molecular structure in a latent space in operation 240. In an example, fine tuning of a molecular structure may be performed based on the property information required on the molecular structure implemented in the latent space. For this, a target score may be determined. In an example embodiment, in order to determine the target score, instruction related to property prediction derived based on information included in the database may be applied. The instruction may be performed based on information stored in the database, and predictions may be implemented based on a stored material and its property. Further, observation information may be provided for deriving a target score. The observation information may include at least one of target screening data and target activity information.

By performing tuning on the input compound in the latent space based on the information provided in this way, a structure of the compound having the desired property may be determined.

Decoding molecular structures determined in the latent space may be performed in operation 250.

Accordingly, the restructured compound may be derived in operation 260.

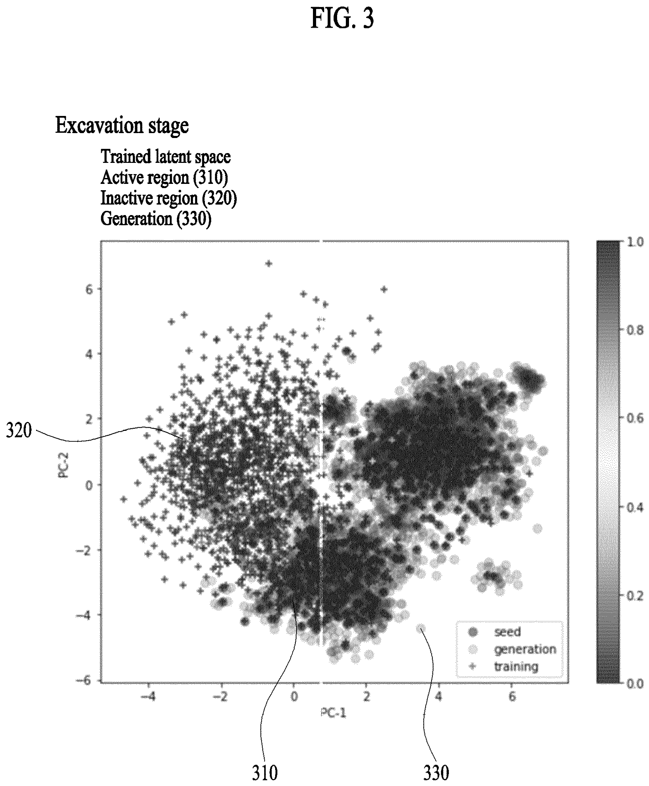

FIG. 3 is a diagram for explaining a compound discovery phase.

FIG. 3 illustrates the discovery phase for deriving a compound in a latent space.

Information associated with an active region 310, an inactive region 320, and a generated part 330 may be determined through comparison of material property and existing data in a latent space. Through the information, a new compound having desired property may be derived.

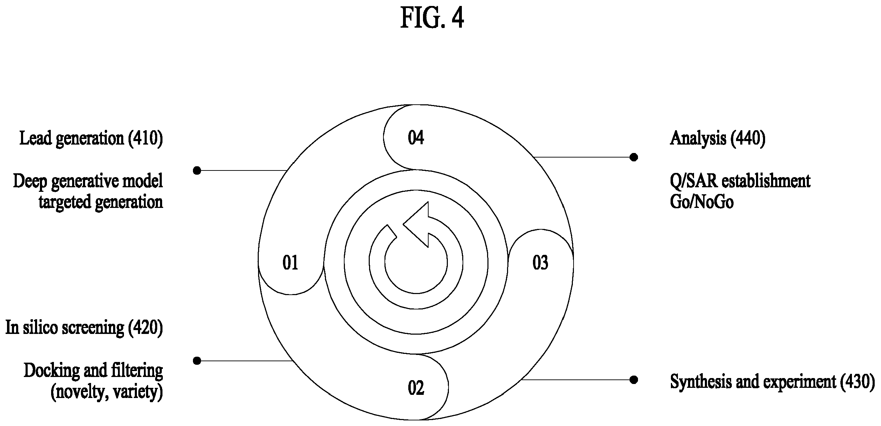

FIG. 4 is a diagram for explaining a compound generation and an analysis process according thereto.

FIG. 4 illustrates the compound generation and analysis process thereof.

The computing apparatus may perform lead generation in operation 410. The generation procedure may be performed through a deep generative model, and through the procedure, target lead generation may be performed.

The computing apparatus may perform screening on the computing apparatus through docking and filtering on the generated lead in operation 420. The procedure may be performed based on at least one of existing database and a learning result, and through the screening procedure, various materials with new structures may be derived.

Screening may allow synthesis and testing of selected materials in operation 430. The syntheses and tests may be performed in a laboratory, and information about experimental results may be provided to the computing apparatus.

Analysis of the experimental result information may be performed in operation 440. The analysis may be performed through methods such as Q/SAR establishment and Go/NoGo.

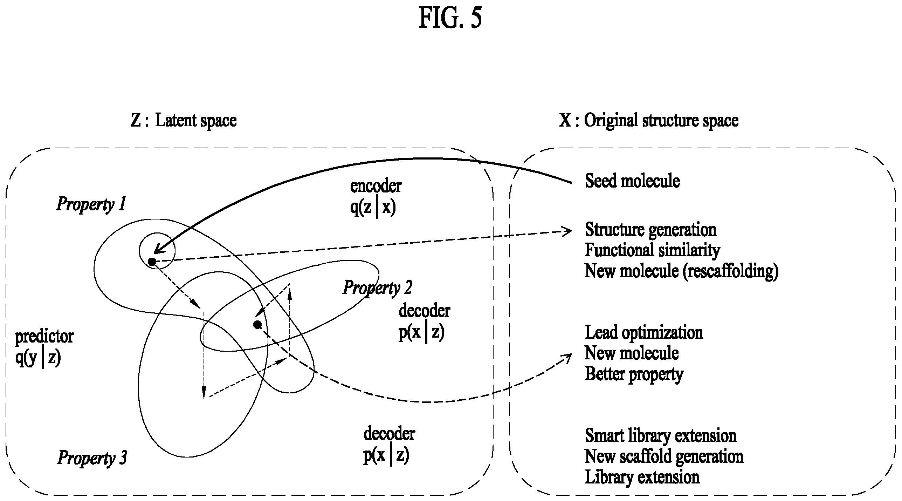

FIG. 5 is a diagram for explaining a relationship between a latent space and an original structure space for compound generation.

FIG. 5 illustrates the relationship between original structure space X and latent space Z. In this case, prediction may be performed based on desired property information Y in the latent space.

A seed molecule in an original structure space may be modified into a latent space, and the computing apparatus may perform encoding such that at least one property is added in the latent space. In addition, a molecule generated in the latent space may be predicted based on desired property information y.

Further, the computing apparatus may generate a material having a new structure with functional similarity in the latent space.

Further, a new molecule with better property may be determined through lead compound optimization based on latent space process.

In this way, a decoder may be used to transform the material generated in the latent space into an original structure space.

Smart libraries may be expanded, such as by generating new scaffolds based on materials generated through such a structure.

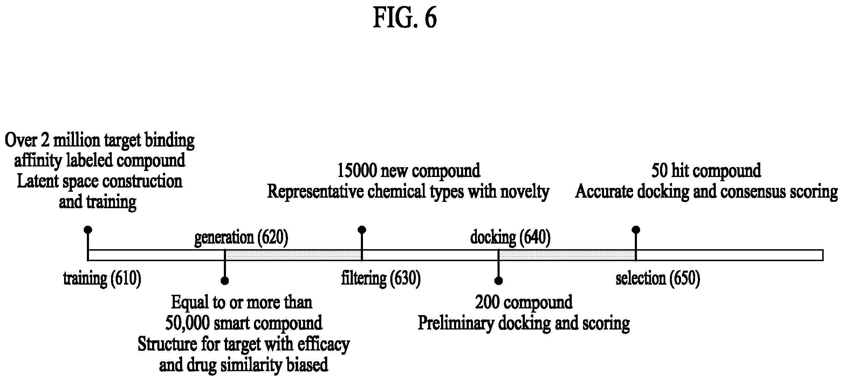

FIG. 6 is a diagram for explaining procedures performed in a process of generating a molecule.

FIG. 6 illustrates the procedures performed in the molecular generation process and the number of target compounds in each procedure.

The computing apparatus may learn existing data about a compound in operation 610. It may establish a latent space based on information associated with equal to or more than two million target-binding affinity-labeled compounds and perform training on the information.

The computing apparatus may generate a structure for a target with a biased efficacy and pharmacological properties with respect to a target material based on trained information, and accordingly, information of equal to or more than 50,000 compounds may be generated in operation 620.

The computing apparatus may perform filtering in consideration of at least one of synthesis difficulty, similarity to an existing material and compatibility with a target protein in operation 630. Accordingly, equal to or more than 15,000 compounds may be selected.

The computing apparatus may perform analysis through preliminary docking of selected compounds, and through this, proceed scoring for the plurality of compounds through compound property information in operation 640. More specifically, a compound satisfying a target property value may have a high score. In this way, about 200 compounds may be selected, and scoring may proceed for the selected compounds.

Accurate docking process for selection may be performed in operation 650, and compounds having a desired property may be more accurately classified through consensus scoring. Through this, about 50 hit compounds may be derived.

Meanwhile, it is obvious that the numbers stated with regard to the example embodiments are exemplary, and an example embodiment of the present disclosure may be implemented in a manner that satisfies at least one of the change direction of the number and the change degree.

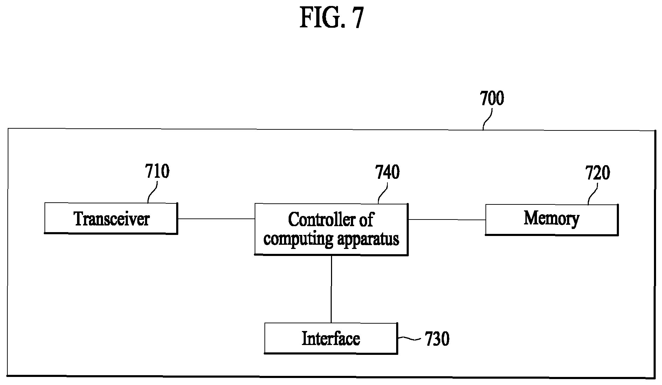

FIG. 7 is a block diagram for explaining a computing apparatus in which a method according to example embodiments of the present disclosure is executed.

FIG. 7 illustrates the computing apparatus according to example embodiments of the present disclosure.

A computing apparatus 700 may include at least one of a transceiver 710, a memory 720, an interface 730 and a controller 740.

The transceiver 710 may perform communication between the computing apparatus 700 and an external node. The transceiver 710 may transmit and receive information through communication, and may be connected to separate database to receive related information.

The memory 720 may store at least one of information related to an operation of the computing apparatus 700 of the present disclosure and information transmitted or received through the transceiver 710.

The interface 730 may be used to input information into the computing apparatus 700. More specifically, at least one of a case in which a user designates a parameter value and a case in which information related to an experiment result is input may be performed through the interface 730.

The controller 740 may control the overall operation of the computing apparatus 700, and the controller 740 may control the computing apparatus 700 to perform a procedure described as operations of the computing apparatus 700 throughout the present disclosure.

New drug development is largely divided into a research phase in which drug materials are discovered and synthesized at the laboratory level, and a development phase in which clinical trials are approved. Among these, the research phase goes through target identification, target validation, screening (target to hit), deriving a lead compound (hit to lead) and optimizing the lead compound (lead optimization). The phase of target identification is determining a target protein to develop a medicine for a specific disease, and whether to proceed is decided by comprehensively assessing the results of previous studies and strategy conformity. The phase of target validation is verifying the correlation and causality between controlling a selected target and a target disease treatment. At this stage, animal test results may also be reviewed using various genomic information, information associated with transgenic animals and materials for research. The phase of screening is the task of finding a material that controls the selected target, and the phase of deriving a lead compound is narrowing the scope by finding the similarity of the structure of compounds obtained as a result of the screening. The phase of optimizing the lead compound is more intensively optimizing the selected compounds series, and hundreds of compounds are tested in vitro and in vivo.

Meanwhile, given its data-driven nature, AI driven drug discovery requires more data from multiple sources, independent predictions and strategic rules than ever before. In particular, a compound design using a generative model, activity prediction, physical property calculation, data processing, analysis, machine learning, and web service development are all required for the production-level protocol of the initial drug discovery project. Thus, a lot of data is required for this.

To meet the expectations, the AI-based new drug discovery platform according to an example embodiment of the present disclosure generates molecules optimized with a scalable distribution system architecture. This example embodiment may be used to generate novel and initially hypothetical hit compounds active against a specific target protein and with desirable drug-likeness, as well as generating visual reports in an automated and scalable manner.

In an actual situation of drug development, multiple parameter optimization (MPO) is required, and thus it is difficult to derive a single methodology that best predicts a goal. Further, since screening rules are usually designed by developers rather than computers, this may become a bottleneck in extending the workflow of new drug discovery.

Selection of synthetic candidates from hypothetical ligands in the drug development phase is based on the screening rules. The screening rules are difficult to be designed in a scalable manner, and a predictive model is needed to systematically select a valid candidate group. Further, the selected synthetic candidate group is used for selection of hypothetical molecules or hypothetical ligands through experiments and verification. In this case, in order for the system to automatically utilize only necessary information in the feedback stage, an integrated architecture is needed.

As described above, conventionally, user intervention for selection and feedback was repeatedly required through multiple pseudo-processes, it was difficult to automate the development phase, and it was difficult to receive feedback information from results. To resolve this, the concept of "dark factory" was introduced into molecular development. The dark factory refers to an unmanned factory without human intervention, and refers to a computer-automated process. When the dark factory is applied to drug discovery, candidate molecules may be generated without intermediate intervention of human experts.

A deep generative workflow may be utilized to automate the drug discovery process. The drug discovery may automate selection and feedback process and increase the accuracy of scoring through the deep generative workflow. Specifically, a score may be calculated by predicting the preference of a compound in the selection and predicting the weight at which the preference value contributes to the score. Further, it can be proceeded to the next step in the order of the compound with the high score. The preference may be determined as an item that may be intuitively obtained from an experimental result. For example, intuitive items such as "Synthesis went well / There was a problem with synthesis," "It was okay in terms of toxicity / There was a problem in terms of toxicity" or "It was good in terms of efficacy / The effect of the drug not good" may be used.

In the feedback step, when there is large difference between a predicted value of the preference and an experimental value, a preference prediction model may be updated. New experimental data may be incorporated into the feedback process by learning the difference in preference results through a machine learning model or a deep learning model and updating the model. The mode of operation may be similarly applied to new compounds.

Each step of the deep generative workflow operates in an integrated way. First, new compound information is input, and common preference $r^{(t)}$ may be predicted through Equation 1 below using the previous preference and the input new information.

$$[\text{Equation 1}]$$
$$r^{(t)} = r^{(t-1)} \cdot \sigma\left(f\left(x^{(t)}\right)\right)$$

Here, $r^{(t-1)}$ is the previous preference, and $x^{(t)}$ is new information.

Next, a compound may be selected by predicting selection score $s^{(t)}$ through Equation 2 based on the preference.

[Equation 2]

$$s^{(t)} = \sigma\left(g\left(r^{(t)}\right)\right)$$

After a compound is selected, feedback is performed through experimentation and verification. In this case, there may be difference between preference $\hat{r}$ for the target drug and preference $r$ of the molecule composed of the selected compound. In addition, based on difference $\mathscr{L}$, feedback may be performed.

[Equation 3]

$$\mathscr{L} = \sum d\left(r, \hat{r}\right) + s \sum (1 - \hat{r}) + (1 - s) \sum \hat{r}$$

Equations 1 to 3 above are example embodiments and are for showing the relationship between each of components, and example embodiments are not limited to the above equations.

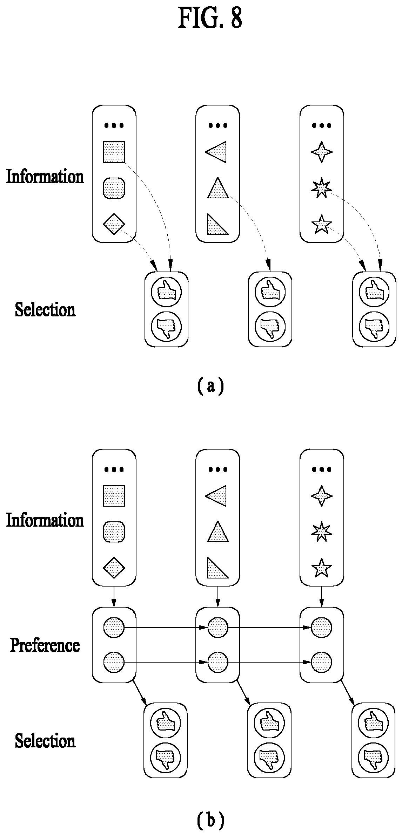

FIG. 8 is a conceptual diagram illustrating a process of discovering a candidate molecule. In FIG. 8, (a) indicates an existing method, and (b) indicates a method to which the deep generative workflow is applied. Conventionally, a user directly designed each compound, and reflected it in a compound selection procedure. However, after the deep generative workflow is applied, preference such as synthesizability, potency and druggability may be evaluated for a discovered compound and reflected in selection. In addition, these selections may continue to be updated automatically based on feedback.

The new drug discovery platform may automate molecule generation and data processing with a scalable distribution system architecture based on in-silico simulation. Specifically, the new drug discovery platform may perform a design related to a molecular structure based on a learning result on a biological activity-related structure, and may select a hit material by post filtering through comparison with an existing structure.

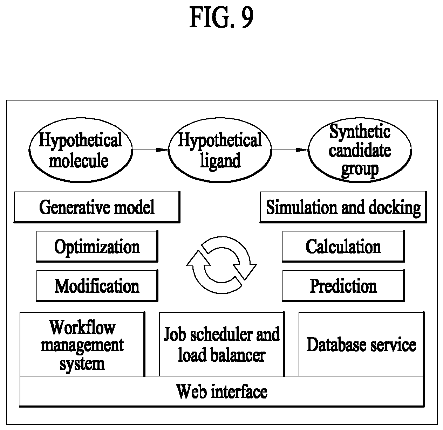

FIG. 9 is a conceptual diagram illustrating an operation of a new drug discovery platform. The new drug discovery platform basically consists of a process of selecting a synthetic candidate group from a hypothetical ligand derived from a hypothetical molecule. In addition, through a generative model, lead compound optimization and modification of compounds may be performed, and calculation and prediction may be performed through simulation and docking. The process may be done automatically through computer simulation, which is an in-silico method.

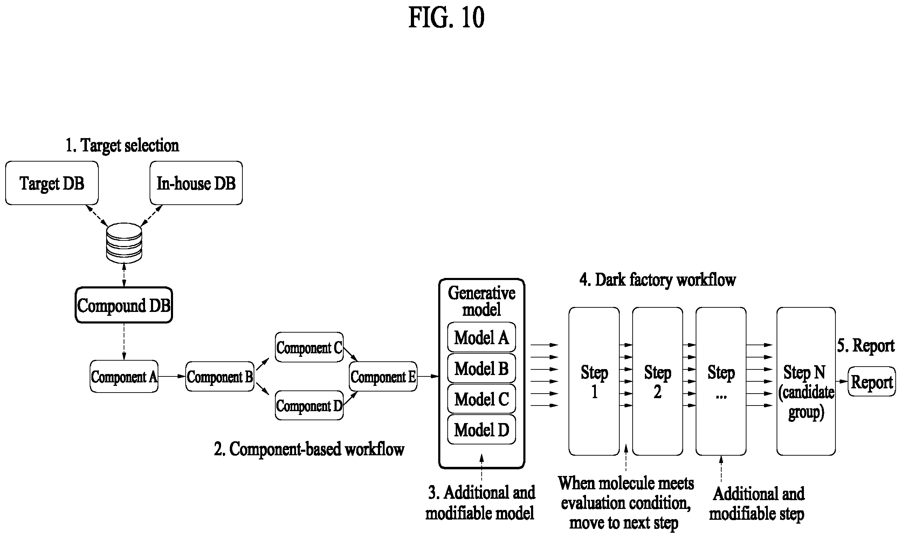

More specifically, a workflow of the new drug discovery platform will be described with reference to FIG. 10. FIG. 10 is a conceptual diagram illustrating a configuration and workflow of a new drug discovery platform.

Referring to FIG. 10, first, a target may be selected from database related to a target protein. The target protein database may include target DB, in-house DB and compound DB. In addition, the generation of a new compound through a workflow composed of component-type pre-processing functions may proceed. In this case, a user may set up the workflow directly for the process. Next, using generative models that may be plugged-in, with generated compounds, candidate materials may be selected through a conveyor belt factory-style deep generative workflow that produces candidate materials through various evaluation methods without human intervention (i.e., in the "dark factory" method). According to an example embodiment, the dark factory workflow may be efficiently changed and improved according to a purpose using a neural network model, and the user may set the workflow directly. The dark factory workflow may consist of a plurality of addable/pluggable steps, and may be used in such a way that each step moves to the next step if a compound meets criteria for evaluation. The evaluation condition at this time may indicate a case where the preference and selection score discussed above with respect to FIG. 8 are satisfied. Finally, a report may be issued for the candidate materials selected in this way and used in the feedback process.

Meanwhile, the series of process for discovering a new compound is repeatedly performed. Specifically, in the process of generating a modified molecule, a latent vector space of a generative model using a genetic algorithm may be searched, and molecules may be generated according to various scores for new scaffolds generated as a result of the search. This is called iterative exploration, and the iterative exploration is a very efficient search method compared to batch search. The batch search is a method of scoring tens to hundreds of thousands of compounds within one cycle, and one cycle may take a very long time due to the large number of target compounds. On the other hand, the iterative exploration is a method of generating and scoring a certain amount of compounds, and the scoring result may be determined in every cycle. That is, a docking score may be determined for each cycle, and an improvement direction may be determined as the cycle progresses. For example, the direction of generation of a compound may be determined in a direction in which the average score for each cycle increases or in a direction in which the number of satisfied compounds increases. A residual vector between the mean hypothetical vector coordinates may be considered to determine the direction of generation of a compound.

A docking score may use a scoring function well known in the art (for example, Schrodinger's glide docking score, autoDock-vina's docking score, similarity between negative volume of panther's binding pocket and 3D structure volume of ligand, and shape similarity of OpenEye ROCS). The docking scores may be considered one by one, or if resources are available, they may be derived by using the ensemble result of each scoring function. According to an example embodiment, a filter may be used to move on to the next cycle if a value passing a filter meets a threshold. Such filters may include a ligand efficiency score, a PAINS filter, a Lilly filter, a chiral center count and an ADMET score. Since the iterative screening produces and scores a certain amount of the compounds, typically one cycle may be completed within one day.

Further, in the lead compound optimization, which is a process of new drug development, a change of structure of a hit compound is often associated with modification of a specific part of a molecule. To this end, optimization is performed by one part of the molecule being fixed and the rest of the structure being modified. However, since the molecule is composed of various partial structures, the part to be modified may be much larger than the fixed part.

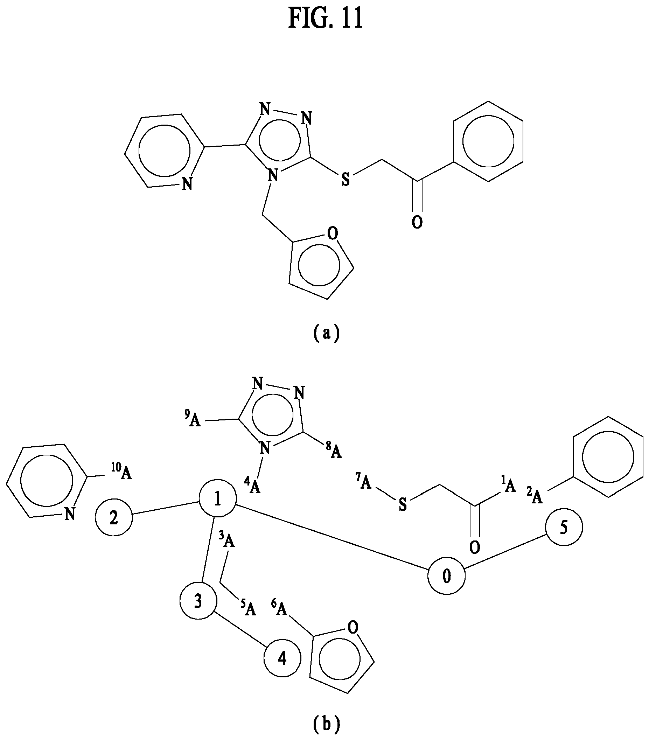

FIG. 11 illustrates an example embodiment of configuring a partial structure tree.

Referring to FIG. 11, the structure (a) is the proposed original molecular structure. If the structure (a) is divided into partial structures, it may be divided into six parts. If the structure (a) is divided into six partial structures and the connection relationship between the partial structures is represented by a solid line, the connection relationship may be expressed in a tree form as shown in (b). Herein, a method of dividing into partial structures and a method of expressing a tree form will be described later.

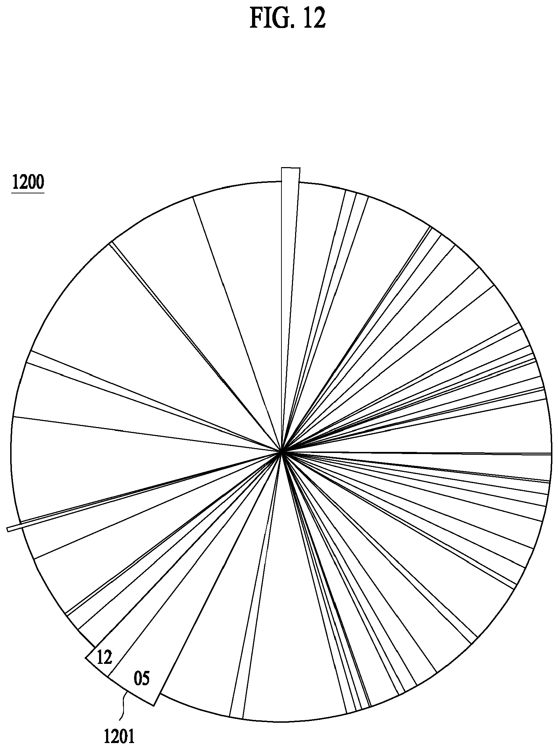

FIG. 12 is a graph showing the pattern distribution of partial structure modification when the sampled molecules of FIG. 11 are modified using an existing generative model.

For example, when indices 0 to 5 are attached to partial structures of the proposed molecular structure of (b) in FIG. 11, "05" with regard to a part 1201 of FIG. 12 indicates that the partial structures of No. 0 and 5 are modified. That is, referring to FIG. 12, in the case of optimizing a lead compound by modifying the partial structures No. 0 and 5 among the molecular structures of (b) in FIG. 11, a structure in which a desired partial structure is modified occupies the small part 1201 of a complete generated structure 1200. For this reason, it may not be easy to optimize a lead compound in a general molecular generative model. Therefore, in order to perform the lead compound optimization more efficiently, a model in which only an interesting part in the molecular structure is changed is required.

The present disclosure proposes a method of generating a sample molecule by modifying an initial molecular structure into smaller partial structures connected to each other and selecting a partial structure to be modified.

In more detail, an original molecular structure may be designed in the form of a tree expressed as partial structures and presented to a user, and a part to be modified may be selected by the user. A partial structure may be variously referred to as a moiety or a subpart.

In the molecular structure designing method according to an example embodiment, the computing apparatus may receive an initial molecular structure, a part to be fixed and a target protein as a parameter. For example, the computing apparatus may receive a target molecular structure, divide the input molecular structure into partial structures, and provide the molecule of which partial structure information is expressed in a tree form to a user. In addition, the computing apparatus may receive information associated with a partial structure desired to be modified from the user. The partial structure may be embedded in a latent space and used to discover similar partial structures. In this case, additional molecular information such as partial charge distribution may be considered. In addition, a molecular sample may be generated based on specified information, and a score may be calculated and output for the generated molecular sample.

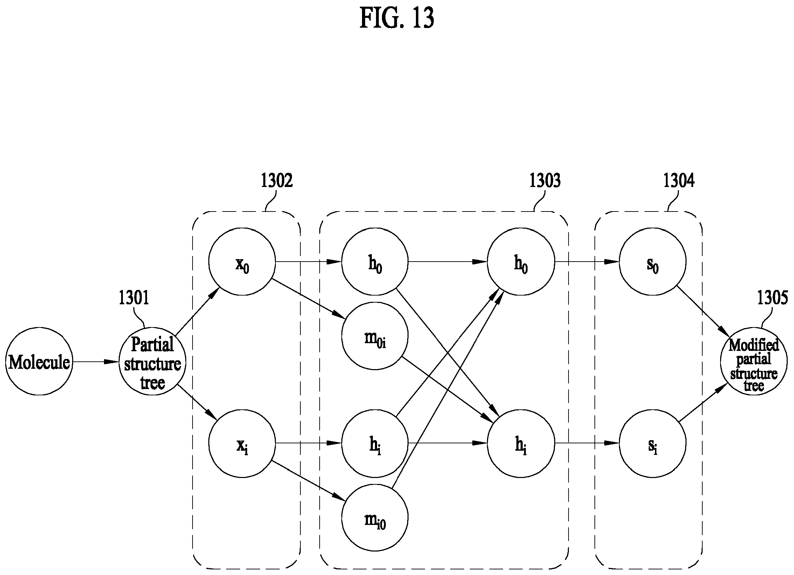

FIG. 13 schematically illustrates a molecular structure designing method according to example embodiments of the present disclosure.

Referring to FIG. 13, a partial structure 1302 to be modified may be selected from a partial structure tree 1301, and the selected partial structure 1302 may be analyzed into various features 1303 through deep learning or machine learning in the computing apparatus to derive a similar partial structure 1304. In addition, the computing apparatus may derive a new molecule by generating a modified partial structure tree 1305 through the similar partial structure 1304. According to an example embodiment, the deep learning or the machine learning may be a model using a graph convolutional network (GCN), and the modified partial structure tree 1305 may be generated through a model using a long short-term memory (LSTM). The molecular structure designing method as shown in FIG. 13 of the present disclosure may be referred to as a moiety-based neural network that may be expressed in a network form.

The molecular structure designing method according to an example embodiment of the present disclosure may generate various partially modified structures while preserving the shape of a given molecule. For example, in designing a molecular structure, what may be considered are a first case considering the combination of all modified partial structures, a second case considering partial structures filtered by the molecular weight so that the size does not differ significantly from an initial molecule, and a third case using the moiety-based neural network according the example embodiment of the present disclosure. The third case may use a neural network model to present all combinations of molecular structures that are different for humans to imagine with few operations. Accordingly, the third case may derive a result in which in a sample space smaller than that of the first case, the average and maximum values of shape similarity with the first molecule are higher than those of the second case. Through this, the molecular structure designing method according to the example embodiments of the present disclosure may efficiently discover molecules similar to an initial molecule.

Further, the method according to an example embodiment of the present disclosure of selectively modifying only a part of a given material structure into a new shape may design a new molecular structure in a way of establishing a learning model that may obtain relationship between partial structures, decomposing a given molecular structure into smaller partial structures linked together, analyzing the partial structures using the learning model and modifying only a desired part of the given molecular structure.

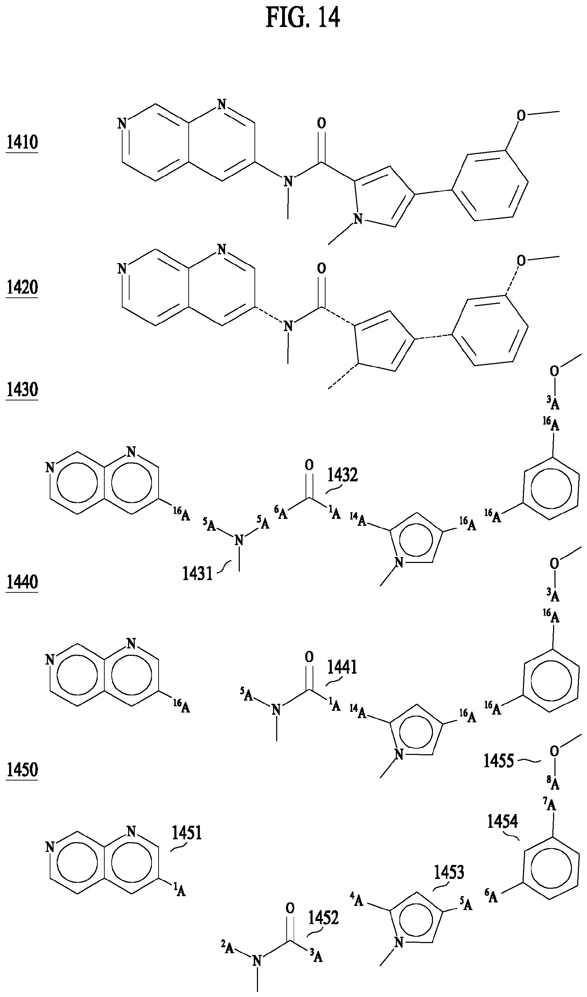

FIG. 14 illustrates a process of decomposing a molecular structure into partial structures according to example embodiments of the present disclosure. First, in order to intuitively grasp an exemplary molecular structure 1410 of a material used in a new drug, the structure is divided into a structure in which one or a number of rings are connected and a chain structure without a ring, and connection between parts may be expressed with dotted lines (reference numeral 1420). In this case, partial structures are generated by mechanically cutting the molecular structure, and a chemically meaningless partial structure may come out. More meaningful partial structures may be obtained by using rules for finding chemically meaningful bonds (for example, RECAP and BRICS), but it may be somewhat different from the intuitive result of decomposition based on a ring structure. Accordingly, as a way to compromise the two, a method of deriving a partial structure that decomposes a molecule based on existing decomposition rules but decomposing the molecule around a ring structure may be considered. Meanwhile, in the description of example embodiments below, each operation may be performed by the computing apparatus.

First, a bond suggested by a kind of rules (for example, BRICS) with respect to the molecular structure (reference numeral 1410) is broken thereby generating a partial structure (reference numeral 1430). As an example, a bonding position of each partial structure is indicated by arbitrary atom A, and the number attached to the bonding position may be a classification number according to the BRICS rule. In addition, when a part not including a ring is divided into a number of pieces, adjacent partial structures may be connected to another (reference numeral 1440). For example, a partial structure 1431 and a partial structure 1432 that do not contain rings are adjacent to each other and thus may be combined into a partial structure 1441. Next, an independent index may be assigned to each position where a binding is broken to indicate the connection relationship (reference numeral 1450).

In order to obtain a clearer ring structure-centered partial structure, when two or more ring structures are included in one partial structure, it may be further separated into a ring and a linker part. Further, when small substituents (for example, 1 to 4 atoms)

remain in a ring structure, the substituents may be further separated from the ring structure.

FIG. 14 illustrates the example of decomposing a molecular structure including a ring structure into partial structures, but through a separation method other than the BRICS separation method, even a molecular structure without a ring structure may be decomposed into partial structures based on other bonding units.

According to an example embodiment, after a molecular structure is decomposed into partial structures, the molecular structure may be represented as a partial structure tree by using the partial structures. Specifically, if each partial structure is expressed as a node, and connection between nodes is expressed as a combination of indices, a partial structure tree that expresses an original structure in the same way may be obtained.

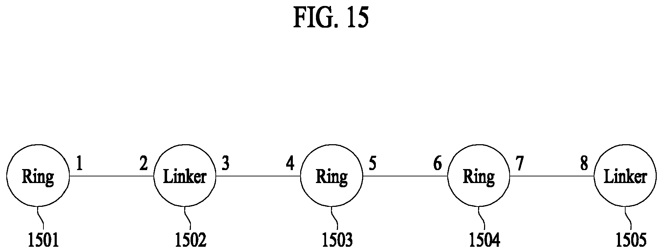

FIG. 15 is a diagram illustrating a partial structure tree according to an example embodiment. As shown by reference numeral 1450 of FIG. 14, a partial structure 1451, a partial structure 1452, a partial structure 1453, a partial structure 1554 and a partial structure 1455 are structures of a ring, a linker, a ring, a ring and a linker in sequence, and binding relationships between the partial structures may be sequentially represented by indices 1, 2, 3, 4, 5, 6, 7 and 8. Accordingly, referring to FIG. 15, the partial structures 1451, 1452, 1453, 1454 and 1455 are represented by a node 1501, a node 1502, a node 1503, a node 1504 and a node 1505, respectively, and thus it may be determined that the connection between each node is expressed as an index. Meanwhile, according to the decomposition of the partial structure as in the example embodiment, there may be one or less than one linker between a ring structure and a ring adjacent to each other.

According to an example embodiment, by learning molecular structures known in the technical field of the present disclosure, a deep learning model obtaining a relationship between partial structures may be generated. Similar partial structures may be selected by extracting information associated with partial structures from molecular structure data and learning the relationship between the partial structures using the deep learning model.

According to an example embodiment, a deep learning model may be configured using an encoder-decoder structure that takes a structure as an input and structure-related information as an output. For example, a model such that an encoder encodes a partial structure fingerprint of a molecular structure (for example, Extended Connectivity FingerPrint (ECFP)) as input, and then a decoder decodes the encoded result to output a string representation (for example, Simplified Molecular-Input Line-Entry System (SMILES)) of a molecular structure.

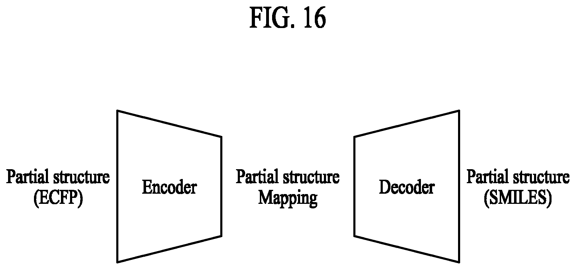

FIG. 16 is a diagram illustrating an encoder-decoder model according to an example embodiment.

An encoder-decoder model may be trained by extracting information associated with a partial structure from data of numerous molecules. The encoder-decoder learning model may be generated by performing learning based on ECFP information (input) and SMILES information (output) thereof for a partial structure known from the example embodiments. Through the learning model, similar molecular structures may have similar mapping values on the mapping obtained by passing the encoder. By encoding partial structure information based on the learning model, mapping information may be obtained, and by decoding the mapping information, SMILES information of the partial structure may be obtained.

Further, in an example embodiment, the computing apparatus may store mapping information for a known partial structure based on the learning model in a storage. In addition, in the example embodiment, if a specific structure is encoded using the learning model, the mapping information of the corresponding partial structure may be determined. And, a similar partial structure may be determined based on the stored mapping information, or mapping information for a modified partial structure may be generated by modifying a part of the generated mapping information, and when the mapping information is decoded, partial structure information thereof may be newly determined.

Further, in an example embodiment, a mapping value may be obtained by encoding a partial structure to be modified or encoding partial structures of known molecular structures. Through this, a model may be obtained that generates new modified partial structures on mapping obtained through an encoder, or selects modified partial structures that are easy to synthesize by comparing mapping values of known molecular structures with mapping values of target partial structures.

According to an example embodiment, using the encoder-decoder model, a new partial structure may be generated by randomly modifying a mapping value obtained by encoding a partial structure to be modified. In this case, the mapping value may indicate a vector value of the target partial structure, and the mapping structure may be changed by applying noise to the vector value. Therefore, by sampling near the position where an existing partial structure is mapped, a new partial structure that is similar but did not exist before is designed. In the example embodiment, adding noise may include adding an arbitrary value to at least one element among elements of the vector value.

Further, using the encoder-decoder model, similar partial structures may be selected by measuring the similarity with previously known partial structures using a mapping value. Mapping values obtained by encoding previously known partial structures through the encoder-decoder model are generated, and a mapping value is generated by encoding a target partial structure to be modified. In addition, by comparing the mapping values, among the previously known partial structures, a partial structure(s) having a similar mapping value is presented as a candidate(s) for the partial structure to be modified.

The encoder-decoder model of the present disclosure may receive a vector value in a matrix from as an input value for a partial structure. Further, in addition to a fingerprint input, the encoder-decoder model of the present disclosure may utilize various methods for expressing a molecular structure, such as input in the form of a graph of a molecular structure or input in the form of a point cloud with coordinate values. Further, in a case of an encoder, models such as deep neural networks, graph convolutional networks and 3D convolutional neural networks may be used according to an input. Further, recurrent neural network models for obtaining a character string representation or other graph generative models may be utilized as a decoder.

The models may utilize methods used to generate a complete molecular structure, and since the size of each partial structure is small, learning may be more effective compared to learning a complete structure. As an example, when configuring a model for simple property prediction, rather than configuring a model by configuring 8,192-dimensional ECFP for a molecular structure of a complete structure, configuring a 512-dimensional ECFP for each partial structure may be used to indicate less errors when using the above encoder (for logP prediction, mean absolute error (MAE) of the model using the complete structure is 0.354, and the MAE of the model using a partial structure is 0.316).

Meanwhile, a partial structure similar to a specific partial structure of a given structure may be obtained through the partial structure learning model according to an example embodiment, but it may be difficult to obtain a similar structure of a desired shape depending on the format of input or output information. For example, in the SMILES string expression, an aromatic hexagonal ring is expressed as "c1ccccc1" and an aliphatic hexagonal ring is expressed as "C1CCCCC1." Since both of the rings are the same hexagonal ring, the aromatic hexagonal ring and the aliphatic hexagonal ring may be viewed as similar materials, but there may be a problem in learning to lose similarity because of the large difference in string representation. To solve this problem, in an example embodiment, the encoder-decoder model may be modified to allow the decoder to predict additional structure properties.

According to an example embodiment, a model that predicts topology information of a partial structure may be added. Topology information is to express only the shape of how atoms are bonded, ignoring information associated with specific types of atoms or bonding types in a molecular structure, and all atoms may be expressed as C and all bonds may be expressed as single bonds. In this case, in the above example embodiment, the aromatic and aliphatic rings had the same topological structure, C1CCCCC1. By allowing the learning model to additionally predict the information, on the mapping obtained through the encoder, it may be learned that materials with similar topological information is mapped to similar positions.

Further, in addition to the topology, a model that predicts the molecular weight may be added so that partial structures of similar scale are assessed to be somewhat similar.

According to the present disclosure, a plurality of partial structures may be encoded thereby mapping values thereof being generated, and a partial structure having a mapping value similar to that of the partial structure to be modified may be selected based on the generated mapping values. Further, the model may generate a mapping value of the partial structure to be modified, and may newly generate a modified partial structure by modifying at least one mapping value among the generated mappings values. In the example embodiment, modifying some values may include modifying values of at least one element among the generated mapping values, and a method of adding or subtracting a value of 1 to 15% of the value of the at least one element and decoding it to newly generate a partial structure may be used.

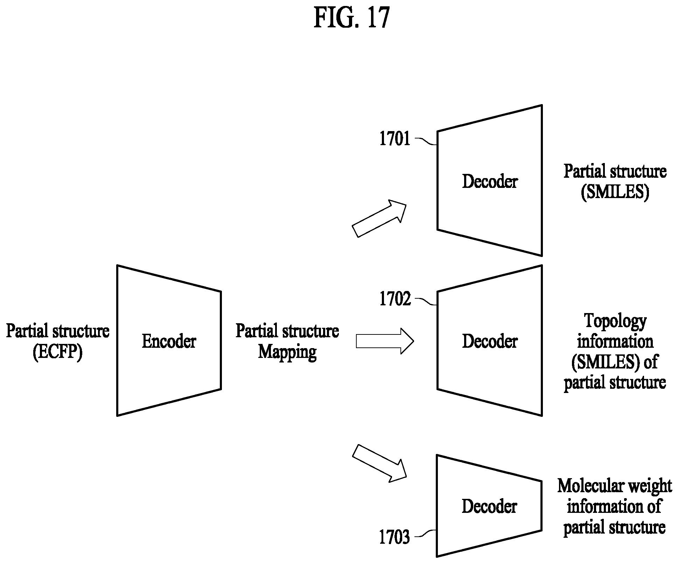

FIG. 17 is a diagram illustrating an encoder-decoder model for further outputting topology information and molecular weight information of a partial structure according to an example embodiment.

According to FIG. 17, the encoder-decoder model of the present disclosure may construct a model to simultaneously predict composition information, topology information and molecular weight information of a partial structure from an encoded result. That is, the learning model may include at least one of a decoder 1701 for outputting a character string expression (SMILES) of a partial structure, a decoder 1702 for outputting topology information of a partial structure in the character string expression (SMILES), and a decoder 1703 for outputting molecular weight information of a partial structure. In addition, through this, more various types of similar structures may be obtained.

The method according to the example embodiment may modify only a desired part of a molecular structure using the encoder-decoder model obtained by learning partial structures of previously known molecular structures. For this, a desired part of a given molecular structure may be recognized, and in this process, an input may be received from a user.

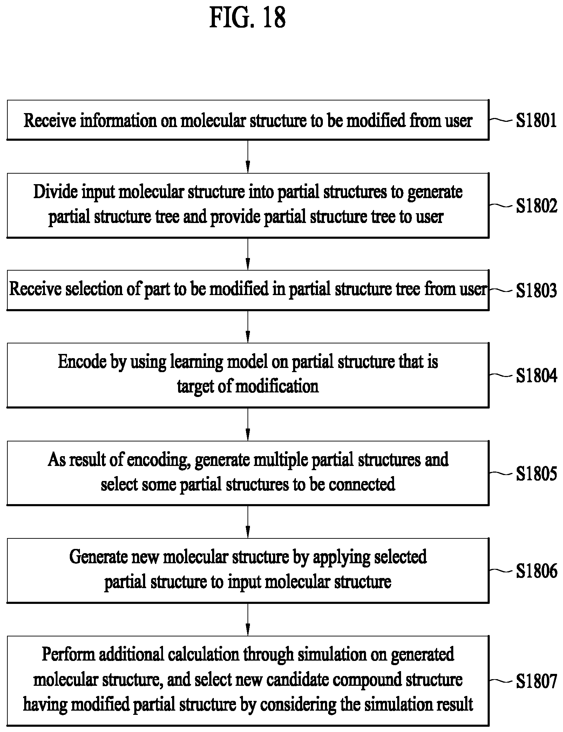

FIG. 18 is a flowchart of a method of modifying a partial structure of a molecular structure according to an example embodiment.

Referring to FIG. 18, in order to modify a partial structure according to the example embodiment, the computing apparatus may receive information about a molecular structure to be modified from a user (S1801). In this case, the user may directly input a character string expression of the molecular structure or may input information by calling information from database in which molecular structures are stored.

Further, the computing apparatus may generate a partial structure tree by dividing the input molecular structure into partial structures and provide the partial structure tree to the user (S1802). In this process, the partial structure division method of the present disclosure described above may be applied. The partial structure tree may be expressed in a tree form with the partial structures as nodes and provided to the user as shown in FIG. 15. In addition, an input for selecting a part to be modified in the partial structure tree may be received from the user (S1803). In the example embodiment, it is described that a part desired to be modified in the partial structure tree is selected and input by the user, but the present disclosure is not limited thereto, and the computing apparatus may adaptively select a partial structure to be modified by referring to a structure of a molecule that has been previously modified.

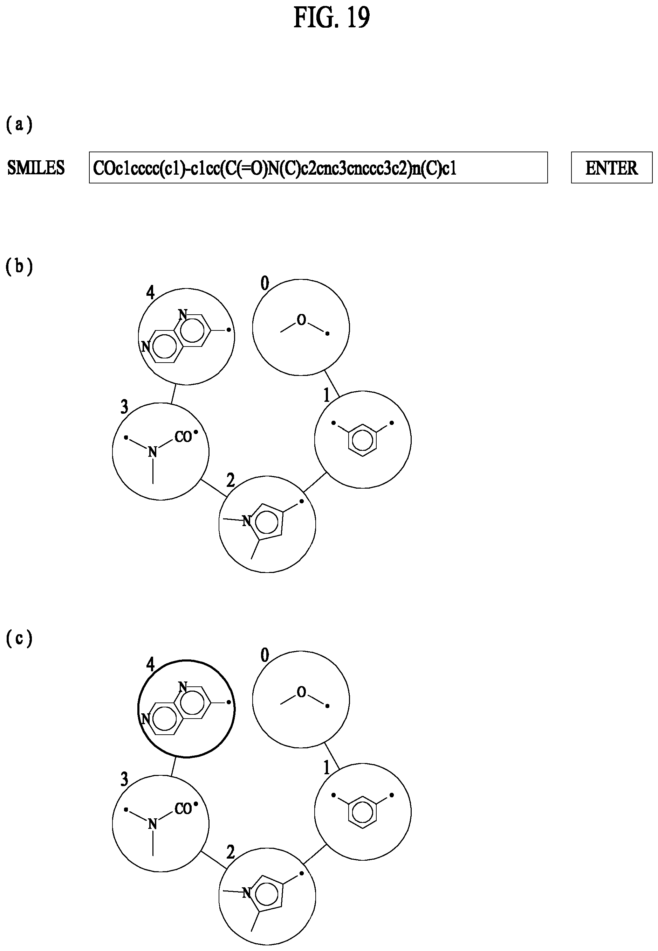

FIG. 19 is a diagram illustrating an example embodiment in which a source molecule to be modified and a partial structure are input. For example, referring to (a) of FIG. 19, a user may input a molecular structure to be modified in the form of a character string expression (SMILES). Then, the system may divide the input molecular structure into partial structures to form a tree, and then output a partial structure tree as shown in (b) of FIG. 19. The user may select a partial structure to be modified among the nodes, that is, the partial structures. (c) of FIG. 19 is an example embodiment illustrating that the user selects node 4 as a target partial structure to be modified.

Next, the computing apparatus may encode the partial structure to be modified using the learning model (S1804). In this case, the encoder-decoder model described above may be applied. A plurality of partial structures may be generated as a result of encoding, and some partial structures may be selected as binding targets (S1805). According to an example embodiment, a new partial structure may be designed by arbitrarily modifying mapping values obtained by encoding the target partial structure by small amounts. Alternatively, by comparing a mapping value of a target partial structure with mapping values obtained by encoding the previously known partial structures, partial structures having close values among the previously known partial structures may be suggested as modification candidates. The former method is useful when generating a structure with greater novelty, and the latter method is useful when generating a structure with easy synthesis. The computing apparatus may select some partial structures so that partial structures of various forms as much as possible may be included in the result according to the example embodiment.

Further, the computing apparatus may generate a new molecular structure by reflecting a selected partial structure to an input molecular structure (S1806). In addition, the computing apparatus may perform additional calculations through simulation on the generated molecular structure, for example, prediction and calculation of efficacy using docking simulation, morphology similarity, pharmacological and toxicity-related efficacy, and the computing apparatus may select a new candidate material structure in which a partial structure is changed by aggregating the simulation result (reference numeral S1807).

According to an example embodiment, after generating a modified partial structure (reference numeral S1805), in order to increase efficiency in the subsequent process, the computing apparatus may go through a process of selecting a partial structure based on a representative structure so as not to include similar structures repeatedly as much as possible. In general, from the chemical point of view, what influences the interaction of a partial structure with surrounding partial structures may be viewed as the distribution of electrons within the partial structures. Therefore, the computing apparatus may select more diverse partial structures by using distribution of electrons to classify partial structures in detail based on similarity of shapes, and select partial structures corresponding to various detailed classifications.

According to the example embodiment, for this purpose, the computing apparatus may first calculate the distribution of electrons associated with the generated partial structure. In this case, as a method of calculating the distribution of electrons associated with partial structures, the Gasteiger partial charge calculation method which is calculating distribution of electrons using electronegativity information may be considered. Alternatively, electronic structure calculations may be performed through quantum mechanics simulation and results thereof may be utilized. Next, the computing apparatus may quantify how many electrons are packed or missing in each atom by summarizing the distribution of electrons obtained as a result of the calculation on an atomic basis. In addition, the computing apparatus may mark a scaffold of a partial structure about an atom with or without electrons to some extent or more. Meanwhile, after the computing apparatus obtains a scaffold including partial charge information for each molecular structure (that is, a partial charge scaffold), and the computing apparatus may exclude a structure in which a partial charge scaffold overlaps to other structure from various partial structures.

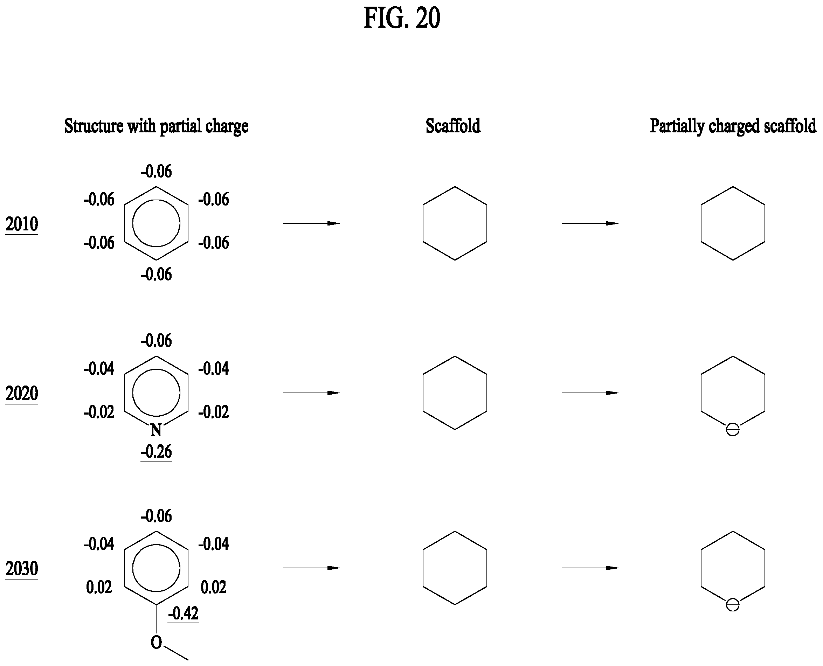

FIG. 20 is a diagram illustrating a partial charge scaffold for an exemplary partial structure. FIG. 20 illustrates an exemplary configuration for a partial charge scaffold.

It may be advantageous to select structures with different partial charge scaffolds to each other, rather than to select similar (or identical) partial structures in terms of a partial charge scaffold, in selecting more diverse partial structures. Structures with different partial charge scaffolds may include, for example, hexagonal ring structures containing two negative charges or a positive charge. For reference, in the case of a structure such as the partial structure 2030 of FIG. 20 to which a substituent is attached, the sum of partial charges of atoms of the substituent may be considered as the partial charge of the position where the substituent is attached on the scaffold. Referring to FIG. 20, three partial structures have a similar scaffold, and in the aspect of the partial charge scaffold, the partial structure 2020 and the partial structure 2030 appear to be similar. Accordingly, rather than selecting the partial structures 2020 and 2030, the method of the present disclosure may select structures with different partial charge scaffolds to each other, for example, the partial structure 2010 and the partial structure 2020 or the partial structure 2010 and the partial structure 2030.

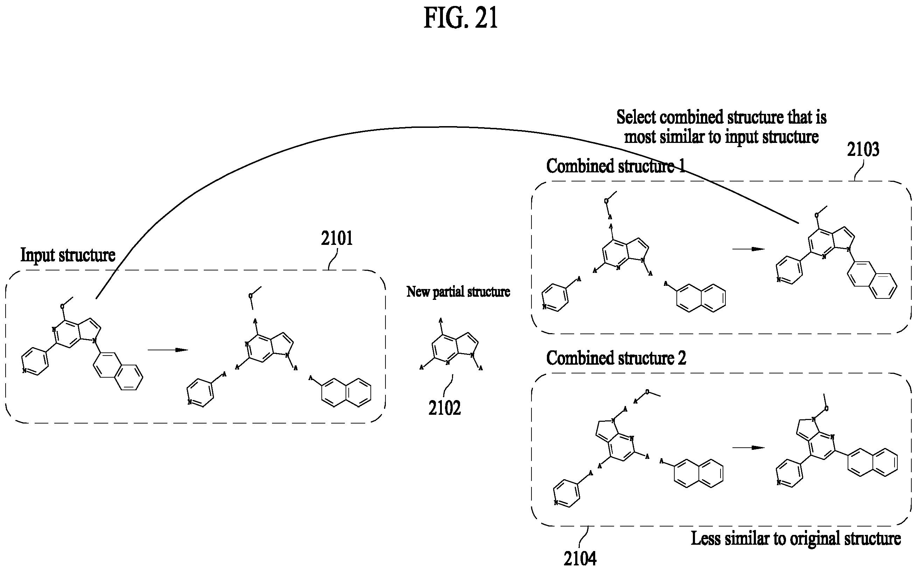

FIG. 21 is a view illustrating a case that may come out in the process of recombining a new partial structure 2102. A new molecule may be generated by designing the new partial structure 2102 based on an input original structure 2101 and by recombination based thereon. In this case, it may be determined at which position the partial structure 2102 is bound, but since there is no index information, which part is connected to the corresponding binding site may not be known. Accordingly, a combined structure 2103 having a shape similar to the input original structure 2101 may be derived, but a combined structure 2104 having lower similarity to the original structure 2101 may be derived. Therefore, in this case, among the combined structures, the combined structure 2103, which is the most morphologically similar to the original structure 2101, is selected. However, since the encoder-decoder model basically learns each partial structure information independently, information associated with the complete structure may not be obtained, and thus in order to select a structure most similar to the original structure, a separate model with a role similar to the above models must be separately trained. Therefrom, compound discovery efficiency and overall consistency may decrease. Therefore, for more efficient decoding, the encoder structure of the model presented above may be configured in a form that may learn both a complete structure and a partial structure at the same time (for example, hierarchical graph convolutional networks).

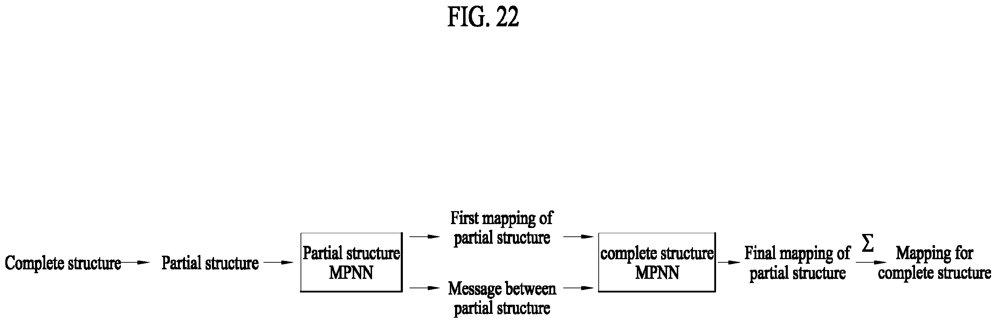

FIG. 22 is a flowchart illustrating an encoder model learning a complete structure and a partial structure according to an example embodiment.

FIG. 22 illustrates generating a learning model that may divide complete structures of known molecules into partial structures, and accordingly may generate mapping information for a complete structure based on message information between partial structures. Specifically, by dividing a complete structure of a molecule into partial structures and encoding each partial structure, first mapping information for a partial structure may be derived. Further, at least one of the derived first mapping information for the partial structure and message information for each partial structure may be encoded to generate final mapping information of the partial structure, and based on the sum of the values of each node (mapping values of individual partial structures), a final mapping value for a complete structure may be generated.

By generating the learning model for a complete structure, in an example embodiment, mapping values of known complete molecular structure are compared to mapping values of a complete molecular structure to which a modified partial structure that is modified when a part of a partial structure constituting a source molecule is applied, and the mapping values may be used to select a molecular structure with high similarity to the source molecular structure. When the learning model is used, the partial structure may be modified, and a mapping value of each complete structure according to a binding method of the modified partial structure may be determined, and by comparing mapping values of the respective complete structures with mapping values of the complete structure of the source molecule, the modified overall structure with a mapping value similar to that of the source molecule may be determined. Through the comparison, a new overall structure having an effect corresponding to that of the source molecule may be easily generated by considering not only the modification of the partial structure but also a bonding method.

According to an example embodiment, a first mapping value for a partial structure may be obtained by dividing a complete structure of a known molecule into partial structures and encoding the plurality of each partial structure. In this case, each partial structure may be expressed and input in the form of a graph, and an encoder may perform encoding by using a message passing neural networks (MPNN) model. A second (final) mapping value of a partial structure may be obtained by encoding the first mapping values obtained by encoding the plurality of partial structures and message information from a binding site of partial structures. In this case, the message information coming from the binding site may indicate the sum of the message information coming through an atom attached to the bonding position of the partial structures.

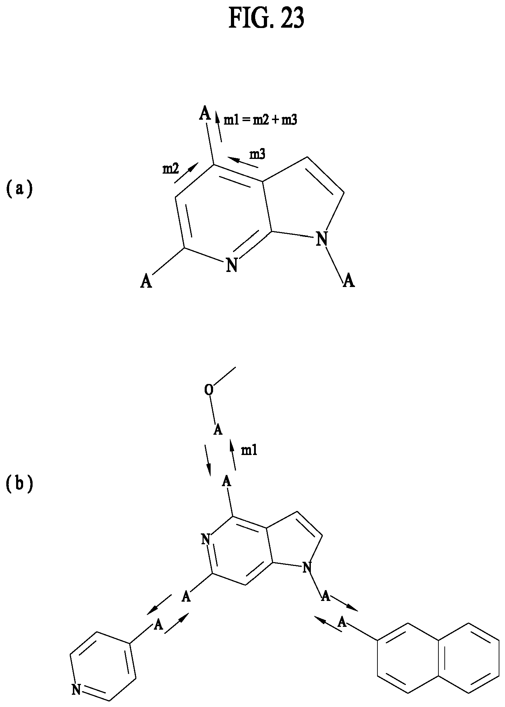

FIG. 23 is a diagram illustrating message information of a partial structure, and referring to (a) of FIG. 23, messages m2 and m3 come from neighboring atoms to binding site (A) of partial structures. By using messages m2 and m3, message information m1 for an adjacent partial structure may be calculated. Referring to (b) of FIG. 23, it may be determined that message information is sent out and received for each binding site.

Returning to FIG. 22, a first mapping value of a partial structure and message information in the high-level graph of partial structures may be encoded. In this case, the encoder may use the MPNN model, just like the previous encoders. As a result of encoding, final mapping values of individual partial structures are derived, and the sum of the mapping values of them become a mapping value of a complete molecular structure. Accordingly, using the model of FIG. 22, not only the mapping values of the individual partial structures but also different mapping values of the complete structure may be obtained according to various cases of reconstructing the partial structures, and thus modifying and reconstructing partial structures may be performed with one model without the need of training a separate model.

After obtaining a new partial structure according to the example embodiments of the present disclosure, the computing apparatus may reconstruct the complete molecular structure. At this time, since information about where a binding is made in partial structures remains, rebinding may occur around the position. However, unlike an original structure, a newly obtained partial structure does not contain information of determining which binding site is binding whereto (that is, index information pasted to a binding site of the original structure), and thus a process of matching it to the original structure is required.

According to an example embodiment of the present disclosure, the computing apparatus may select a structure having a close result value (for example, a latent variable value obtained by encoding a complete molecular structure) by comparing results of the encoding the complete molecular structure using the model of FIG. 22 to results of encoding various reconstructed structures that may be generated according to the combination of binding sites with the same model. That is, one model may select a structure with high similarity to the original structure among various reconstructed structures by using the model of FIG. 22, which encodes the complete structure into one latent variable (vector) to obtain a mapping value of the complete structure.

Meanwhile, complete structures generated through the above process may be filtered through additional simulations. For example, materials may be selected for testing first, which are predicted to be excellent by using performance prediction using docking, comparison of shape similarity in space with existing materials, and prediction results for various performance related to drug properties.

Example embodiment 1

In order to verify the method of generating compound information according the example embodiments of the present disclosure, the computing apparatus may perform a search for a new material by modifying a part of a main reaction part of an existing material into various forms. The performance of a target existing material is at the level of IC50 44 nM, and changes for improvement may be attempted in a critical reactive part of the material. For this, four sets may be formed by setting the number of partial structures that may be modified in the material to about one to three. In addition, 100 partial charge scaffolds may be selected in consideration of similar structures for each partial structure to be modified for each set. A partial structure to be modified may be selected with a sampling method by setting the partial structure having a higher frequency of appearance in the existing data to have a higher probability for each scaffold. In fact, each set may be made by sampling 1 million partial structures in this way. For 1 million materials, shape similarity with an existing structure may be calculated, and 10,000 materials with high similarity may be selected. By predicting drug properties and toxicity using predictive models for the selected 10,000 materials, and performing docking simulations on target proteins, the top 100 materials that are expected to show excellent performance may be finally selected and synthesized. At this time, synthesis is carried out on 30 types of materials that are easy to synthesize, and 12 types of materials may have significantly improved performance (< 10 nM) compared to the existing types of materials.

Example embodiment 2

Through the method of generating compound information according the example embodiments of the present disclosure, some important partial structures may be determined. Further, when modifying a molecular structure to optimize other properties of a target molecule, the computing apparatus may try modification after specifying and inputting another part not including an important part. Through this, a material having excellent properties other than the corresponding performance may be produced while maintaining the performance of the target existing material. In order to improve the reaction selectivity of a material that has secured performance for a target protein to some extent, a modified structure may be generated by adding a partial structure expected to affect the selectivity, and other partial structures that may be used in addition to the added partial structure may be added so that the search for new materials may proceed.

Example embodiment 3

By constructing a fixed part and a modifiable part in various ways through the method of generating compound information according to the example embodiments of the present disclosure, and by analyzing a changing pattern of the predicted performance, a partial structure that has a significant impact on the performance of the compound may be specified. If there is a significant change in the performance of a compound when a partial structure is changed to a similar form, the part may have a significant impact on the performance. On the other hand, if there is no significant change in performance, the partial structure may be determined as a part that has no significant relationship with performance. By using this, the processor may automatically select a partial structure and generate a modified structure without a user having to go through a process of inputting a partial structure, and thus larger volumes of automated materials may be detected.

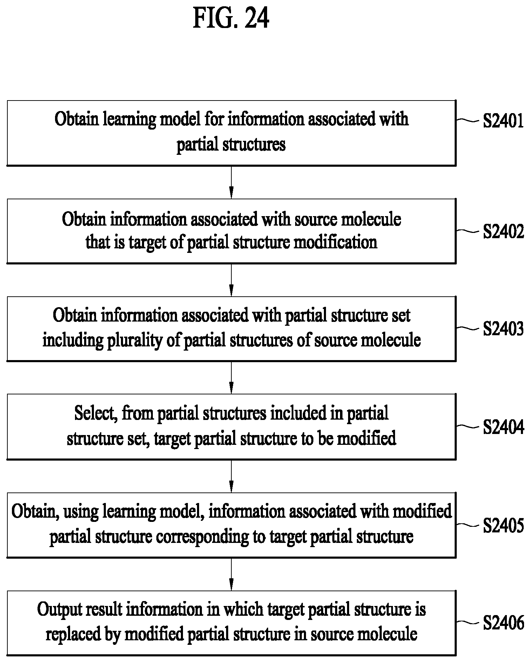

FIG. 24 is a flowchart illustrating a method of generating compound information according to an example embodiment.

According to the method of generating compound information according to the example embodiments of the present disclosure, the computing apparatus may obtain a learning model based on information associated with a partial structure (reference numeral S2401). For example, information associated with a partial structure may be extracted from data of a large number of known molecules, and the computing apparatus may configure a learning model that learns relationship between partial structures by learning the information associated with the partial structures. The learning model may be configured as an encoder-decoder structure.

Then, the computing apparatus may obtain information associated with a source molecule that is a target of partial structure modification (reference numeral S2402). For example, the computing apparatus may receive a character string expression of a molecule from a user or recognize molecular information stored in database. According to an example embodiment, a source molecule may include at least one ring structure.

Next, the computing apparatus may obtain information associated with a partial structure set including a plurality of partial structures of the source molecule (reference numeral S2403). Information of a partial structure set may be generated by dividing a molecular structure included in the source molecule into a first subset of partial structures including at least one ring structure and a second subset of partial structures not including a ring structure, and by assigning indices to binding sites of partial structures. At this time, the number of partial structures that are from the second subset of partial structures positioned between adjacent partial structures included in the first subset of partial structures may be less than or equal to one. The information of a partial structure set may include partial structure tree information indicating a connection between nodes using an assigned indices, after setting each of the partial structures included in the first subset of partial structures and the partial structure included in the second subset of partial structures as a node.

Next, the computing apparatus may select a target partial structure to be modified from among partial structures of a partial structure set (reference numeral S2404). The learning model may be a model obtained by learning information about partial structures of a plurality of previously known molecules. According to an example embodiment, information associated with the target partial structure to be modified among the partial structures of the partial structure set may be obtained based on information input by a user based on the partial structure tree information.

The computing apparatus may utilize the learning model to obtain information associated with a modified partial structure for the target partial structure (reference numeral S2405). Result information in which the modified partial structure is applied to the target partial structure in the source molecule may be output (reference numeral S2406).

The computing apparatus may analyze (encode) the information associated with the target partial structure using the learning model to obtain information associated with the modified partial structure for the target partial structure, and may generate a first mapping value of the target partial structure as a result of the analysis. Further, the computing apparatus may arbitrarily change the first mapping value to generate a second mapping value, and the computing apparatus may decode the generated second mapping value to obtain information associated with the modified partial structure. In particular, the information associated with the modified partial structure may include one or more of composition information associated with the modified partial structure, topology information associated with the modified partial structure, and molecular weight information associated with the modified partial structure. In this case, the learning model may be a deep learning model, and an encoder-decoder model may be applied. Further, the encoder may utilize the hierarchical graph convolutional networks model.

Meanwhile, in order to obtain various information associated with a modified partial structure, the computing apparatus may calculate distribution of electrons associated with the modified partial structure, generate a partial charge scaffold, and select a partial structure with different partial charge scaffolds to each other from the modified partial structures.

Further, the computing apparatus may determine a mapping value of a target partial structure in order to obtain information associated with the modified partial structure with respect to the target partial structure, and may determine, based on the learning model, one or more mapping values associated with at least one known partial structure. In addition, the modified partial structure may be determined from among at least one known partial structure based on the mapping value of the target partial structure and at least one of the mapping values. For example, the mapping value of the target partial structure may be compared to at least some of the mapping values derived by encoding general partial structures used to generate the learning model, and as a result of comparison, partial structures having similar mapping values may be presented as modified partial structures.

According to an example embodiment, in order to combine the modified partial structure into a structure similar to a structure of a source molecule, the computing apparatus may learn relationship information between partial structures of previously known molecules. The trained learning model may be configured as an encoder-decoder model. In order to determine information of at least one partial structure of the partial structure set, the computing apparatus may represent a plurality of partial structures of the partial structure set in graph form, perform first encoding based on the plurality of partial structures expressed in the graph form, and as a result, generate first mapping values of the plurality of partial structures. Further, the computing apparatus may perform second encoding on the first mapping values and message information of each of the plurality of partial structures, and as a result, the computing apparatus may generate the second mapping values of the plurality of partial structures. The message information may indicate relationship between a partial structure and an adjacent partial structure. Further, the computing apparatus may calculate an overall mapping value of the molecular structure based on the second mapping values. In this case, the first encoding and the second encoding may utilize the MPNN model, and the overall mapping value may be the sum of each of the second mapping values of the partial structures. Further, an input of the encoder may be various forms. A method of representing 0 and 1, a method of representing an integer, and a node/connection relationship matrix including categorical/real number information in the case of a graph may be utilized. In an example embodiment, the partial structure information input to the encoder may be a vector or matrix composed of 0 and 1, and a partial structure mapping value may be a vector or a matrix having a real number. The dimension of a partial structure may be a higher dimension than the dimension of a mapping value.

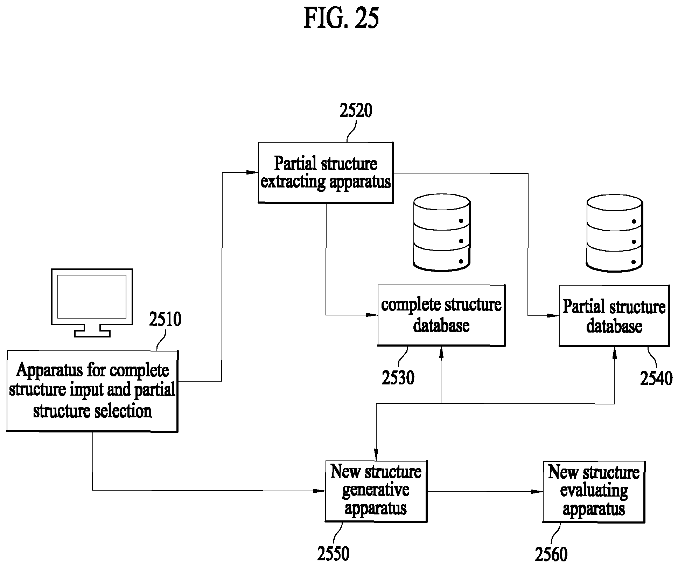

FIG. 25 is a diagram schematically illustrating a system for generating compound information according to an example embodiment.

Referring to FIG. 25, a system of generating compound information may include an apparatus for complete structure input and partial structure selection 2510, a partial structure extracting apparatus 2520, a complete structure database 2530, a partial structure database 2540, a new structure generative apparatus 2550 and a new structure evaluating apparatus 2560.

The apparatus for complete structure input and partial structure selection 2510 may receive information associated with a source molecule from a user, output information associated with a plurality of partial structures included in the source molecule, and receive an input for selecting a target partial structure to be modified.

The partial structure extracting apparatus 2520 may divide the received source molecule information into a plurality of partial structures. The information of the source molecule may be stored in the complete structure database 2530, and the plurality of partial structures divided therefrom may be stored in the partial structure database 2540. According to an example embodiment, the partial structure extracting apparatus 2520 may divide a molecular structure included in the source molecule into a first subset of partial structures including at least one ring structure and a second subset of partial structures not including a ring structure, and assign indices to binding sites of partial structures. In this case, the number of partial structure that are from the second subset of partial structures positioned between partial structures included in the first subset of partial structures may be less than or equal to one. Further, the information of the plurality of partial structures may be partial structure tree information indicating a connection between nodes using an assigned indices, after setting each of the partial structures included in the first subset of partial structures and the partial structure included in the second subset of partial structures as a node. If a ring structure is contained, the molecular structure may be divided around the ring structure, but otherwise, the molecular structure may be divided around a specific structure.

The apparatus for complete structure input and partial structure selection 2510 may receive information associated with a source molecule from a user, output information associated with a plurality of partial structures included in the source molecule, and receive an input for selecting a target partial structure to be modified.

The new structure generative apparatus 2550 may obtain information associated with the modified partial structure of the target partial structure using the learning model, and may output information as a result of applying the modified partial structure to the target partial structure from the source molecule. According to an example embodiment, the learning model may be obtained by learning a plurality of previously known partial structures, and may include information associated with a relationship between partial structures.

According to an example embodiment, obtaining information of a modified partial structure by the new structure generative apparatus 2550 may include encoding information of a target partial structure using the learning model, and generating a first mapping value of the target partial structure as a result. Further, the new structure generative apparatus 2550 may arbitrarily change the first mapping value to generate a second mapping value, and obtain information associated with the modified partial structure by decoding based on the second mapping value. In this case, the information associated with the modified partial structure may include composition information, topology information and molecular weight information associated with the modified partial structure.

In order to obtain more diverse partial structures, the new structure generative apparatus 2550 may calculate distribution of electrons associated with a modified partial structure, generate a partial charge scaffold based on the calculated distribution of electrons, and select a partial charge scaffold in a modified partial structure with different partial charge scaffolds to each other.

Further, the new structure generative apparatus 2550 may analyze the partial structures divided from the source molecule using the learning model to obtain a partial structure that is easily connected to the source molecule, and present it as a candidate for a modified partial structure. The new structure generative apparatus 2550 may determine a mapping value of a target partial structure, and may determine at least one mapping value among mapping values of a plurality of previously known partial structures by using the learning model. Further, a modified partial structure may be determined from at least one known partial structure based on the mapping value of the target partial structure and at least one of the mapping values. For example, by comparing the mapping value of the target partial structure to the at least one of mapping values, partial structures with high similarity may be presented as candidates for the modified partial structure.

According to an example embodiment, obtaining the learning model used in the new structure generative apparatus 2550 may include expressing a plurality of partial structures in graph form, performing first encoding based on the plurality of partial structures expressed in the graph form, and as a result, generating first mapping values of the plurality of partial structures. Further, the first mapping values and message information of the plurality of partial structures may be second encoded, and as a result, second mapping values of the plurality of partial structures may be generated. In this case, the message information indicates a relationship between a partial structure and an adjacent partial structure. Further, an overall mapping value of the molecular structure may be calculated based on the second mapping values, and the overall mapping value may be the sum of the second mapping values of each of the plurality of partial structures.

The new structure evaluating apparatus 2560 may perform additional calculations through simulation on a molecular structure generated by the new structure generative apparatus 2550. Further, a new candidate material structure with a modified partial structure may be selected by aggregating the simulation result. In addition, the new structure evaluating apparatus 2560 may filter the generated molecular structure through additional simulation.

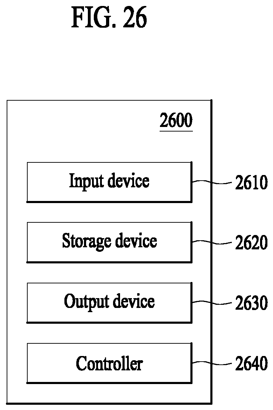

FIG. 26 is a block diagram illustrating a computing apparatus for generating compound information according to an example embodiment of the present disclosure.

Referring to FIG. 26, a computing apparatus 2600 of generating compound information may include an input device 2610, a storage 2620, and an output device 2630 and a controller 2640. The controller 2640 may obtain a learning model for information associated with partial structures, obtain information associated with a source molecule that is a target of partial structure modification and obtain information associated with a partial structure set including a plurality of partial structures of the source molecule. Further, the learning model may be used to obtain information associated with the modified partial structure for a selected target partial structure, and output information as a result of applying the modified partial structure to the target partial structure from the source molecule.

According to an embodiment, the learning model may be obtained by learning a plurality of previously known partial structures, and may include information associated with relationship between partial structures.

According to an example embodiment, controller 2640 obtaining information of a partial structure set may include dividing a molecular structure included in the source molecule into a first subset of partial structures including at least one ring structure and a second subset of partial structures not including a ring structure, and assigning indices to binding sites of partial structures. In this case, the number of partial structure that are from the second subset of partial structures positioned between partial structures included in the first subset of partial structures may be less than or equal to one. If a ring structure is contained, the molecular structure may be divided around the ring structure, but otherwise, the molecular structure may be divided around a specific structure. The information associated with the partial structure may be partial structure tree information indicating a connection between nodes using an assigned indices, after setting each of the partial structures included in the first subset of partial structures and the partial structure included in the second subset of partial structures as a node. According to an example embodiment, controller 2640 may output partial structure tree information through the output device 2630, and may receive an input of a partial structure to be modified based on the partial structure tree information through the input device 2610.

According to an example embodiment, obtaining the information of the modified structure by the controller 2640 may include analyzing the information associated with the target partial structure using the learning model, and generating a first value of the target partial structure as a result. Further, when the controller 2640 obtains information associated with a modified partial structure, the first mapping value may be arbitrarily changed to generate a second mapping value, and performing decoding based on the second mapping value to obtain information associated with the modified partial structure. In this case, the information associated with the modified partial structure may include composition information associated with the modified partial structure, topology information associated with the modified partial structure and molecular weight information associated with the modified partial structure.

In order to obtain more diverse partial structures, the controller 2640 may calculate distribution of electrons associated with the modified partial structure, generate a partial charge scaffold based on the calculated distribution of electrons, and select a partial structure with different partial charge scaffolds to each other from the modified partial structures.

According to an example embodiment, the controller 2640 may analyze partial structures divided from previously known molecules using the learning model and present the divided partial structures as candidates for a modified partial structure. The controller 2640 may determine a mapping value of a target partial structure, and may determine at least one mapping value among mapping values of a plurality of previously known partial structures by using the learning model. Further, a modified partial structure may be determined from at least one known partial structure based on the mapping value of the target partial s1tructure and at least one of the mapping values. For example, by comparing the mapping value of the target partial structure to at least one of mapping values, partial structures with high similarity may be presented as candidates for the modified partial structure.

According to an example embodiment, the controller 2640 obtaining the learning model for information of a partial structure may include obtaining information associated with overall structures of a plurality of known molecules, expressing partial structures of the complete structures of the plurality of known molecules in graph form, respectively, performing first encoding based on the plurality of partial structures expressed in the graph form and as a result, generating first mapping values of the plurality of partial structures. Further, the controller 2640 may perform second encoding on the first mapping values and message information of each of the plurality of partial structures, and as a result, the computing apparatus may generate the second mapping values of the plurality of partial structures. In this case, the message information may indicate relationship between a partial structure and an adjacent partial structure. Further, the controller 2640 may calculate an overall mapping value of the molecular structure based on the second mapping values, and the overall mapping value may be the sum of each of the second mapping values of the partial structures.

According to an example embodiment, the input device 2610 and the output device 2630 may include the apparatus for complete structure input and partial structure selection 2510, and the storage 2620 may include the complete structure database 2530 and the partial structure database 2540. Further, the controller 2640 may include the partial structure extracting apparatus 2520, the new structure generative apparatus 2550 and the new structure evaluating apparatus 2560.

The models described with reference to FIGS. 25 and 26 are for implementing the example embodiments of the present disclosure, and are not limited to the above-described configurations.

**Claims**

1. An AI-based molecular structure designing method for designing a molecular structure of a new drug candidate, the method performed at a computing apparatus comprising:

    obtaining, at the computing apparatus, a learning model configured as an encoder-decoder structure based on information of a partial structure (S2401), wherein the learning model is obtained by learning information about partial structures of a plurality of previously known molecules;
    obtaining, at the computing apparatus, from a user, information of the molecular structure of a source molecule that is a target of a partial structure modification (S2402);
    obtaining, at the computing apparatus, information of a partial structure set including a plurality of partial structures of the source molecule by dividing the molecular structure of the source molecule into a subset of partial structures (S2403);

selecting, at the computing apparatus, from the partial structures included in the partial structure set, a target partial structure to be modified (S2404), based on information input by the user;

obtaining, at the computing apparatus, using the learning model, information of a modified partial structure corresponding to the target partial structure (S2405); and

outputting, at the computing apparatus, result information of the molecular structure in which the target partial structure is replaced in the molecular structure of the source molecule by the modified partial structure (S2406),

wherein obtaining, at the computing apparatus, using the learning model, the information of the modified partial structure comprises:

generating, at the computing apparatus, a first vector value by encoding the target partial structure;

generating, at the computing apparatus, a second vector value by changing the first vector value; and

obtaining, at the computing apparatus, the information of the modified partial structure by decoding the second vector value;

wherein the information of the modified partial structure comprises one or more of composition information of the modified partial structure, topology information of the modified partial structure, and molecular weight information of the modified partial structure.

2. The method of claim 1, wherein obtaining, at the computing apparatus, the information of the partial structure set comprises:

dividing, at the computing apparatus, a molecular structure included in the source molecule into a first subset of partial structures including at least one ring structure and a second subset of partial structures not including a ring structure; and

assigning, at the computing apparatus, an index to a binding site between partial structures, wherein the binding site is the position at which the partial structure (2102) is bound in the source molecule,

wherein the number of partial structures that are from the second subset of partial structures and positioned between partial structures included in the first subset of partial structures is less than or equal to one.

3. The method of claim 2, wherein the information of the partial structure set comprises partial structure tree information setting each of a partial structure included in the first subset of partial structures and a partial structure included in the second subset of partial structures as a node, and indicating connection between the nodes using the assigned indices, wherein the structure tree is generated by dividing the input molecular structure into partial structures.

4. A computing apparatus (2600) configured to design a molecular structure of a new drug candidate, comprising:

an input device (2610) configured to receive a user input;

a storage device (2620) configured to store information;

an output device (2630) configured to output information; and

a controller (2640) configured to:

obtain a learning model configured as an encoder-decoder structure based on information of a partial structure, wherein the learning model is obtained by learning information about partial structures of a plurality of previously known molecules,

obtain, from a user, information of the molecular structure of a source molecule that is a target of partial structure modification,

obtain information of a partial structure set including a plurality of partial structures of the source molecule by dividing the molecular structure of the source molecule into a subset of partial structures,

select, from the partial structures included in the partial structure set, a target partial structure to be modified based on information input by the user,

obtain, using the learning model, information of a modified partial structure corresponding to the target partial structure, and

output result information of the molecular structure in which the target partial structure is replaced in the molecular structure of the source molecule by the modified partial structure;

wherein, when the controller obtains, using the learning model, the information of the modified partial structure, the controller is further configured to:

generate a first vector value by encoding the target partial structure;

generate a second vector value by changing the first vector value; and

obtain the information of the modified partial structure by decoding the second vector value,

wherein the information of the modified partial structure comprises at least one of composition information of the modified partial structure, topology information of the modified partial structure and molecular weight information of the modified partial structure.

5. The computing apparatus of claim 4,

wherein the controller is configured to divide a molecular structure included in the source molecule into a first subset of partial structures including at least one ring structure and a second subset of partial structures not including a ring structure, and assign an index to a binding site between partial structures, wherein the binding site is the position at which the partial structure (2102) is bound in the source molecule,
wherein the number of partial structures that are from the second subset of partial structures and positioned between partial structures included in the first subset of partial structures is less than or equal to one; and wherein the information of the partial structure set comprises partial structure tree information setting each of a partial structure included in the first subset of partial structures and a partial structure included in the second subset of partial structures as a node, and indicating connection between the nodes using the assigned indices.

**Patentansprüche**

1. KI-basiertes Verfahren zum Entwurf einer Molekularstruktur eines neuen Arzneimittelkandidaten, wobei das Verfahren auf einer Computervorrichtung durchgeführt wird und umfasst:

Erhalten eines Lernmodells, das als eine Codierer-Decodierer-Struktur basierend auf Informationen über eine Teilstruktur eingerichtet ist, auf der Computervorrichtung (S2401), wobei das Lernmodell durch Lernen von Informationen über Teilstrukturen einer Mehrzahl bereits bekannter Moleküle erhalten wird;
Erhalten von Informationen über die Molekularstruktur eines Quellmoleküls, das ein Ziel einer Teilstrukturmodifikation ist, von einem Benutzer auf der Computervorrichtung (S2402);
Erhalten von Informationen über eine Teilstrukturmenge einschließlich einer Mehrzahl von Teilstrukturen des Quellmoleküls durch Aufteilen der Molekularstruktur des Quellmoleküls in eine Teilmenge von Teilstrukturen auf der Computervorrichtung (S2403);
Auswählen einer zu modifizierenden Zielteilstruktur aus den in der Teilstrukturmenge enthaltenen Teilstrukturen basierend auf vom Benutzer eingegebenen Informationen auf der Computervorrichtung (S2404);
Erhalten von Informationen über eine modifizierte Teilstruktur, die der Zielteilstruktur entspricht, unter Verwendung des Lernmodells auf der Computervorrichtung (S2405); und
Ausgeben von Ergebnisinformationen über die Molekularstruktur, in der die Zielteilstruktur in der Molekularstruktur des Quellmoleküls durch die modifizierte Teilstruktur ersetzt ist, auf der Computervorrichtung (S2406),
wobei das Erhalten der Informationen über die modifizierte Teilstruktur unter Verwendung des Lernmodells auf der Computervorrichtung umfasst:

Erzeugen eines ersten Vektorwerts durch Codieren der Zielteilstruktur auf der Computervorrichtung;
Erzeugen eines zweiten Vektorwerts durch Ändern des ersten Vektorwerts auf der Computervorrichtung; und
Erhalten der Informationen über die modifizierte Teilstruktur durch Decodieren des zweiten Vektorwerts auf der Computervorrichtung;

wobei die Informationen über die modifizierte Teilstruktur eine oder mehrere von Zusammensetzungsinformationen der modifizierten Teilstruktur, Topologieinformationen der modifizierten Teilstruktur und Molekulargewichtsinformationen der modifizierten Teilstruktur umfassen.

2. Verfahren nach Anspruch 1, wobei das Erhalten der Informationen über die Teilstrukturmenge auf der Computervorrichtung Folgendes umfasst:

Aufteilen einer im Quellmolekül enthaltenen Molekularstruktur in eine erste Teilmenge von Teilstrukturen, die mindestens eine Ringstruktur enthalten, und eine zweite Teilmenge von Teilstrukturen, die keine Ringstruktur enthalten, auf der Computervorrichtung und
Zuweisen eines Indexes zu einer Bindungsstelle zwischen Teilstrukturen auf der Computervorrichtung, wobei die Bindungsstelle die Position ist, an der die Teilstruktur (2102) im Quellmolekül gebunden ist,
wobei die Anzahl von Teilstrukturen, die aus der zweiten Teilmenge von Teilstrukturen stammen und zwischen in der ersten Teilmenge von Teilstrukturen enthaltenen Teilstrukturen positioniert sind, kleiner oder gleich eins ist.

3. Verfahren nach Anspruch 2, wobei die Informationen über die Teilstrukturmenge Teilstrukturbauminformationen umfassen, die jede von einer in der ersten Teilmenge von Teilstrukturen enthaltenen Teilstruktur und einer in der zweiten Teilmenge von Teilstrukturen enthaltenen Teilstruktur als einen Knoten festlegen und unter Verwendung der zugewiesenen Indizes eine Verbindung zwischen den Knoten angeben,
wobei der Strukturbaum durch Aufteilen der eingegebenen Molekularstruktur in Teilstrukturen erzeugt wird.

4. Computervorrichtung (2600), die zum Entwurf einer Molekularstruktur eines neuen Arzneimittelkandidaten eingerichtet ist und umfasst:

eine Eingabevorrichtung (2610), die zum Empfangen einer Benutzereingabe eingerichtet ist;
eine Speichervorrichtung (2620), die zum Speichern von Informationen eingerichtet ist;
eine Ausgabevorrichtung (2630), die zum Ausgeben von Informationen eingerichtet ist; und
eine Steuerung (2640), die eingerichtet ist zum:

Erhalten eines Lernmodells, das als eine Codierer-Decodierer-Struktur basierend auf Informationen über eine Teilstruktur eingerichtet ist, wobei das Lernmodell durch Lernen von Informationen über Teilstrukturen einer Mehrzahl bereits bekannter Moleküle erhalten wird;
Erhalten von Informationen über die Molekularstruktur eines Quellmoleküls, das ein Ziel einer Teilstrukturmodifikation ist, von einem Benutzer;
Erhalten von Informationen über eine Teilstrukturmenge einschließlich einer Mehrzahl von Teilstrukturen des Quellmoleküls durch Aufteilen der Molekularstruktur des Quellmoleküls in eine Teilmenge von Teilstrukturen;
Auswählen einer zu modifizierenden Zielteilstruktur aus den in der Teilstrukturmenge enthaltenen Teilstrukturen basierend auf vom Benutzer eingegebenen Informationen;
Erhalten von Informationen über eine modifizierte Teilstruktur, die der Zielteilstruktur entspricht, unter Verwendung des Lernmodells, und
Ausgeben von Ergebnisinformationen über die Molekularstruktur, in der die Zielteilstruktur in der Molekularstruktur des Quellmoleküls durch die modifizierte Teilstruktur ersetzt ist;
wobei, wenn die Steuerung unter Verwendung des Lernmodells die Informationen über die modifizierte Teilstruktur erhält, die Steuerung ferner eingerichtet ist zum:

Erzeugen eines ersten Vektorwerts durch Codieren der Zielteilstruktur;
Erzeugen eines zweiten Vektorwerts durch Ändern des ersten Vektorwerts; und
Erhalten der Informationen über die modifizierte Teilstruktur durch Decodieren des zweiten Vektorwerts;

wobei die Informationen über die modifizierte Teilstruktur mindestens eine von Zusammensetzungsinformationen der modifizierten Teilstruktur, Topologieinformationen der modifizierten Teilstruktur und Molekulargewichtsinformationen der modifizierten Teilstruktur umfassen.

5. Computervorrichtung nach Anspruch 4,

wobei die Steuerung eingerichtet ist zum Aufteilen einer im Quellmolekül enthaltenen Molekularstruktur in eine erste Teilmenge von Teilstrukturen, die mindestens eine Ringstruktur enthalten, und eine zweite Teilmenge von Teilstrukturen, die keine Ringstruktur enthalten, und zum Zuweisen eines Indexes zu einer Bindungsstelle zwischen Teilstrukturen, wobei die Bindungsstelle die Position ist, an der die Teilstruktur (2102) im Quellmolekül gebunden ist, wobei die Anzahl von Teilstrukturen, die aus der zweiten Teilmenge von Teilstrukturen stammen und zwischen in der ersten Teilmenge von Teilstrukturen enthaltenen Teilstrukturen positioniert sind, kleiner oder gleich eins ist; und wobei die Informationen über die Teilstrukturmenge Teilstrukturbauminformationen umfassen, die jede von einer in der ersten Teilmenge von Teilstrukturen enthaltenen Teilstruktur und einer in der zweiten Teilmenge von Teilstrukturen enthaltenen Teilstruktur als einen Knoten festlegen und unter Verwendung der zugewiesenen Indizes eine Verbindung zwischen den Knoten angeben.

## Revendications

1. Procédé de conception de structure moléculaire basée sur l'IA permettant de concevoir la structure moléculaire d'un nouveau médicament candidat, le procédé étant exécuté sur un appareil informatique comprenant:

l'obtention, dans l'appareil informatique, d'un modèle d'apprentissage configuré comme une structure d'encodage-décodage basée sur les informations d'une structure partielle (S2401), le modèle d'apprentissage étant obtenu par l'apprentissage d'informations concernant les structures partielles d'une pluralité de molécules déjà connues;

l'obtention, dans l'appareil informatique, provenant d'un utilisateur, des informations sur la structure moléculaire d'une molécule source qui est la cible d'une modification partielle de la structure (S2402);

l'obtention, dans l'appareil informatique, des informations sur un ensemble de structures partielles comprenant une pluralité de structures partielles de la molécule source par division de la structure moléculaire de la molécule source en un sous-ensemble de structures partielles (S2403);

la sélection, dans l'appareil informatique, parmi les structures partielles incluses dans l'ensemble de structures partielles, d'une structure partielle cible à modifier (S2404), sur la base des informations entrées par l'utilisateur;

l'obtention, dans l'appareil informatique, à l'aide du modèle d'apprentissage, des informations sur une structure partielle modifiée correspondant à la structure partielle cible (S2405); et

la sortie, dans l'appareil informatique, des informations de résultat de la structure moléculaire dans laquelle la structure partielle cible est remplacée dans la structure moléculaire de la molécule source par la structure partielle modifiée (S2406),

dans lequel l'obtention, dans l'appareil informatique, à l'aide du modèle d'apprentissage, des informations de la structure partielle modifiée consiste à:

    la génération, dans l'appareil informatique, d'une première valeur vectorielle par codage de la structure partielle cible;

    la génération, dans l'appareil informatique, d'une seconde valeur vectorielle par modification de la première valeur vectorielle; et

    l'obtention, dans l'appareil informatique, des informations sur la structure partielle modifiée par décodage de la seconde valeur vectorielle;

dans lequel les informations sur la structure partielle modifiée comprennent une ou plusieurs parmi des informations sur la composition de la structure partielle modifiée, des informations de topologie de la structure partielle modifiée et des informations sur le poids moléculaire de la structure partielle modifiée.

2. Procédé selon la revendication 1, dans lequel l'obtention, dans l'appareil informatique, des informations de l'ensemble de structures partielles consiste à:

    diviser, dans l'appareil informatique, d'une structure moléculaire incluse dans la molécule source en un premier sous-ensemble de structures partielles comprenant au moins une structure annulaire et un second sous-ensemble de structures partielles ne comprenant pas de structure annulaire; et

    attribuer, dans l'appareil informatique, d'un repère à un site de liaison entre les structures partielles, le site de liaison étant la position à laquelle la structure partielle (2102) est liée dans la molécule source,

    dans lequel le nombre de structures partielles issues du second sous-ensemble de structures partielles et placées entre des structures partielles incluses dans le premier sous-ensemble de structures partielles est inférieur ou égal à un.

3. Procédé selon la revendication 2, dans lequel les informations de l'ensemble de structures partielles comprennent des informations sur l'arbre des structures partielles qui définissent chaque structure partielle incluse dans le premier sous-ensemble de structures partielles et une structure partielle incluse dans le second sous-ensemble de structures partielles comme un nœud, et qui indiquent la liaison entre les nœuds à l'aide des repères attribués,

dans lequel l'arbre de structure est généré par division de la structure moléculaire d'entrée en structures partielles.

4. Appareil informatique (2600) configuré pour concevoir une structure moléculaire d'un nouveau médicament candidat, comprenant :

    un dispositif d'entrée (2610) configuré pour recevoir une entrée utilisateur;

    une mémoire (2620) configurée pour stocker des informations;

    un dispositif de sortie (2630) configuré pour délivrer des informations; et

    un dispositif de commande (2640) configuré pour:

        obtenir un modèle d'apprentissage configuré comme une structure d'encodage-décodage basée sur les informations d'une structure partielle, le modèle d'apprentissage étant obtenu par l'apprentissage d'informations concernant les structures partielles d'une pluralité de molécules déjà connues;

        obtenir, en provenance d'un utilisateur, les informations sur la structure moléculaire d'une molécule source qui est la cible d'une modification partielle de la structure,

obtenir les informations sur un ensemble de structures partielles comprenant une pluralité de structures partielles de la molécule source par division de la structure moléculaire de la molécule source en un sous-ensemble de structures partielles,

sélectionner, parmi les structures partielles incluses dans l'ensemble de structures partielles, une structure partielle cible à modifier, sur la base des informations entrées par l'utilisateur,

obtenir, à l'aide du modèle d'apprentissage, les informations sur une structure partielle modifiée correspondant à la structure partielle cible, et

faire sortir les informations de résultat de la structure moléculaire dans laquelle la structure partielle cible est remplacée dans la structure moléculaire de la molécule source par la structure partielle modifiée;

dans lequel, lorsque le dispositif de commande obtient, à l'aide du modèle d'apprentissage, les informations de la structure partielle modifiée, le dispositif de commande est en outre configuré pour:

générer une première valeur vectorielle par codage de la structure partielle cible;
générer une seconde valeur vectorielle par modification de la première valeur vectorielle; et
obtenir les informations de la structure partielle modifiée par décodage de la seconde valeur vectorielle,

dans lequel les informations de la structure partielle modifiée comprennent une ou plusieurs parmi des informations sur la composition de la structure partielle modifiée, des informations de topologie de la structure partielle modifiée et des informations sur le poids moléculaire de la structure partielle modifiée.

5. Appareil informatique selon la revendication 4,

dans lequel le dispositif de commande est configuré pour diviser une structure moléculaire incluse dans la molécule source en un premier sous-ensemble de structures partielles comprenant au moins une structure annulaire et un second sous-ensemble de structures partielles ne comprenant pas de structure annulaire, et pour attribuer un repère à un site de liaison entre les structures partielles, le site de liaison étant la position à laquelle la structure partielle (2102) est liée dans la molécule source,

dans lequel le nombre de structures partielles issues du second sous-ensemble de structures partielles et positionnées entre des structures partielles incluses dans le premier sous-ensemble de structures partielles est inférieur ou égal à un; et dans lequel les informations de l'ensemble de structures partielles comprennent des informations sur l'arbre de structures partielles définissant chaque structure partielle incluse dans le premier sous-ensemble de structures partielles et une structure partielle incluse dans le second sous-ensemble de structures partielles comme un nœud, et indiquant la liaison entre les nœuds à l'aide des repères attribués.

# FIG. 1

```
        ┌──────────────┐
        │    Start     │
        └──────┬───────┘
               │
               ▼
┌─────────────────────────────────────┐
│ Learn structure related to biological activity │ ── 105
└─────────────────────────────────────┘
               │
               ▼
┌─────────────────────────────────────┐
│ Design and generate molecule structure │
│       based on learning result        │ ── 110
└─────────────────────────────────────┘
               │
               ▼
┌─────────────────────────────────────┐
│      Compare to existing structure      │ ── 115
└─────────────────────────────────────┘
               │
               ▼
┌─────────────────────────────────────┐
│             Post filtering              │ ── 120
└─────────────────────────────────────┘
               │
               ▼
┌─────────────────────────────────────────────┐
│ Active material simulation (in silico screening) │ ── 125
└─────────────────────────────────────────────┘
               │
               ▼
┌─────────────────────────────────────┐
│          Synthesize and test           │ ── 130
└─────────────────────────────────────┘
               │
               ▼
        ┌──────────────┐
        │     End      │
        └──────────────┘
```

# FIG. 2

# FIG. 3

**Excavation stage**

Trained latent space
Active region (310)
Inactive region (320)
Generation (330)

FIG. 4

Lead generation (410)

Deep generative model
targeted generation

In silico screening (420)

Docking and filtering
(novelty, variety)

04

01

03

02

Analysis (440)

Q/SAR establishment
Go/NoGo

Synthesis and experiment (430)

EP 4 050 612 B1

## FIG. 5

Z : Latent space

X : Original structure space

encoder
q(z | x)

*Property 1*

predictor
q(y | z)

*Property 2*

decoder
p(x | z)

*Property 3*

decoder
p(x | z)

Seed molecule

Structure generation
Functional similarity
New molecule (rescaffolding)

Lead optimization
New molecule
Better property

Smart library extension
New scaffold generation
Library extension

EP 4 050 612 B1

# FIG. 6

Over 2 million target binding
affinity labeled compound
Latent space construction
and training

15000 new compound
Representative chemical types with novelty

50 hit compound
Accurate docking and consensus scoring

generation (620)

docking (640)

training (610)

filtering (630)

selection (650)

Equal to or more than
50,000 smart compound
Structure for target with efficacy
and drug similarity biased

200 compound
Preliminary docking and scoring

EP 4 050 612 B1

# FIG. 7

700

| 710 | 740 | 720 |
|-----|-----|-----|
| Transceiver | Controller of computing apparatus | Memory |

Interface —730

# FIG. 8

( a )

( b )

## FIG. 9

# FIG. 10

1. Target selection

Target DB

In-house DB

Compound DB

Component A → Component B → Component C, Component D → Component E

2. Component-based workflow

Generative model

Model A

Model B

Model C

Model D

3. Additional and modifiable model

4. Dark factory workflow

Step 1 → Step 2 → Step ... → Step N (candidate group)

When molecule meets evaluation condition, move to next step

Additional and modifiable step

5. Report

Report

EP 4 050 612 B1

# FIG. 11

(a)

(b)

# FIG. 12

FIG. 13

# FIG. 14

1410

1420

1430

1432

1431

1440

1441

1450

1455

1454

1451

1453

1452

FIG. 15

Ring —1— —2— Linker —3— —4— Ring —5— —6— Ring —7— —8— Linker

1501      1502      1503      1504      1505

# FIG. 16

| Partial structure (ECFP) | Encoder | Partial structure Mapping | Decoder | Partial structure (SMILES) |

# FIG. 17

Partial structure
(ECFP) — Encoder — Partial structure
Mapping

1701 — Decoder — Partial structure
(SMILES)

1702 — Decoder — Topology
information
(SMILES) of
partial structure

1703 — Decoder — Molecular weight
information of
partial structure

# FIG. 18

Receive information on molecular structure to be modified from user ⎯ S1801

Divide input molecular structure into partial structures to generate partial structure tree and provide partial structure tree to user ⎯ S1802

Receive selection of part to be modified in partial structure tree from user ⎯ S1803

Encode by using learning model on partial structure that is target of modification ⎯ S1804

As result of encoding, generate multiple partial structures and select some partial structures to be connected ⎯ S1805

Generate new molecular structure by applying selected partial structure to input molecular structure ⎯ S1806

Perform additional calculation through simulation on generated molecular structure, and select new candidate compound structure having modified partial structure by considering the simulation result ⎯ S1807

# FIG. 19

(a)

SMILES COc1cccc(c1)-c1cc(C(=O)N(C)c2cnc3cnccc3c2)n(C)c1 ENTER

(b)

(c)

# FIG. 20

Structure with partial charge          Scaffold          Partially charged scaffold

2010

-0.06
-0.06      -0.06
-0.06      -0.06
-0.06

2020

-0.06
-0.04      -0.04
-0.02      -0.02
N
-0.26

2030

-0.06
-0.04      -0.04
0.02      0.02
-0.42
O

EP 4 050 612 B1

# FIG. 21

Input structure

2101

New partial structure

2102

Select combined structure that is
most similar to input structure

Combined structure 1

2103

Combined structure 2

2104

Less similar to original structure

# FIG. 22

EP 4 050 612 B1

Complete structure → Partial structure → | Partial structure MPNN | → First mapping of partial structure → | complete structure MPNN | → Final mapping of partial structure $\xrightarrow{\Sigma}$ Mapping for complete structure

| Partial structure MPNN | → Message between partial structure → | complete structure MPNN |

# FIG. 23

(a)

(b)

# FIG. 24

Obtain learning model for information associated with partial structures — S2401

Obtain information associated with source molecule that is target of partial structure modification — S2402

Obtain information associated with partial structure set including plurality of partial structures of source molecule — S2403

Select, from partial structures included in partial structure set, target partial structure to be modified — S2404

Obtain, using learning model, information associated with modified partial structure corresponding to target partial structure — S2405

Output result information in which target partial structure is replaced by modified partial structure in source molecule — S2406

# FIG. 25

# FIG. 26

2600

| Input device | — 2610 |
| Storage device | — 2620 |
| Output device | — 2630 |
| Controller | — 2640 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *Deep Reinforcement Learning for Multiparameter Optimization in de novo Drug Design*, 19 June 2019, vol. 59 (7), 3166-3176
- *De novo design of drug-like molecules by a fragment-based molecular evolutionary approach*, 28 December 2013, vol. 54 (1), 49-56
- *Fragment Informatics and Computational Fragment-Based Drug Design: An Overview and Update*, 19 May 2013, vol. 33 (3), 554-598